# EUROPÄISCHE PATENTSCHRIFT

(11) **EP 2 004 815 B1**
(45) Veröffentlichungstag und Bekanntmachung des Hinweises auf die Patenterteilung: **28.08.2013**
(21) Anmeldenummer: 07727884.4
(22) Anmeldetag: 05.04.2007
(51) Int. Cl.: C12N 9/20, C12Q 1/44, C12Q 1/61, C12N 5/10

(54) **FUNKTIONALE EXPRESSION VON TRIACYLGLYCEROL-LIPASEN**
FUNCTIONAL EXPRESSION OF TRIACYLGLYCEROL LIPASES
EXPRESSION FONCTIONNELLE DE TRIACYLGLYCEROL LIPASES

(30) Priorität: 05.04.2006 DE 102006016023
(43) Veröffentlichungstag der Anmeldung: 24.12.2008
(73) Patentinhaber: BASF SE, 67056 Ludwigshafen (DE)
(72) Erfinder: HAUER, Bernhard, 67136 Fussgönheim (DE); HÄRING, Dietmar, 68535 Neu-Edingen (DE); BRANNEBY, Cecilia, 67069 Ludwigshafen (DE); RUSNAK, Monika, 70199 Stuttgart (DE); LIU, Danni, 70199 Stuttgart (DE); SCHMID, Rolf, 70569 Stuttgart (DE)
(86) Internationale Anmeldenummer: PCT/EP2007/053416
(87) Internationale Veröffentlichungsnummer: WO 2007/113336

(56) Entgegenhaltungen:
- WO-A-2004/024954
- RUSNAK M: "Untersuchungen zur enzymatischen Enantiomerentrennung von Glykolethern und Etablierung neuer Methoden des synthetischen Shufflings" [Online] 2004, DISSERTATION , XP002470263 Gefunden im Internet: URL:http://elib.uni-stuttgart.de/opus/voll texte/2005/2160/pdf/dis_komplett.pdf> das ganze Dokument, insbesondere folgende Kapitel: 5.5.1. (S.84-86); 6.3.5. (S.115,116); 8.2.4. (S.159-162)
- SHARMA R ET AL: "Production, purification, characterization, and applications of lipases" BIOTECHNOLOGY ADVANCES, ELSEVIER PUBLISHING, BARKING, GB, Bd. 19, Nr. 8, Dezember 2001 (2001-12), Seiten 627-662, XP004343846 ISSN: 0734-9750
- MONIKA RUSNAK ET AL: "The Putative Lipase, AF1763, from Archaeoglobus fulgidusis a Carboxylesterase with a Very High pH Optimum" BIOTECHNOLOGY LETTERS, KLUWER ACADEMIC PUBLISHERS, DO, Bd. 27, Nr. 11, 1. Juni 2005 (2005-06-01), Seiten 743-748, XP019230854 ISSN: 1573-6776
- GERRITSE GIJS ET AL: "Development of a lipase fermentation process that uses a recombinant Pseudomonas alcaligenes strain" APPLIED AND ENVIRONMENTAL MICROBIOLOGY, Bd. 64, Nr. 7, Juli 1998 (1998-07), Seiten 2644-2651, XP002470261 ISSN: 0099-2240
- SUEN WEN-CHEN ET AL: "Improved activity and thermostability of Candida antarctica lipase B by DNA family shuffling" PROTEIN ENGINEERING DESIGN & SELECTION, Bd. 17, Nr. 2, Februar 2004 (2004-02), Seiten 133-140, XP002470262 ISSN: 1741-0126
- HOEGH I ET AL: "Two lipases from Candida antarctica: cloning and expression in Aspergillus oryzae" CAN. J. BOT., Bd. 73(Suppl.1), 1995, Seiten S869-S875, XP009096359
- BANEYX F ET AL: "Recombinant protein folding and misfolding in Escherichia coli" NATURE BIOTECHNOLOGY, NATURE PUBLISHING GROUP, NEW YORK, NY, US, Bd. 22, Nr. 11, November 2004 (2004-11), Seiten 1399-1408, XP003009809 ISSN: 1087-0156
- LIU D ET AL: "Functional expression of Candida antarctica lipase B in the Escherichia coli cytoplasm-a screening system for a frequently used biocatalyst" APPLIED MICROBIOLOGY AND BIOTECHNOLOGY, SPRINGER-VERLAG, BE, Bd. 72, Nr. 5, 16. Mai 2006 (2006-05-16), Seiten 1024-1032, XP019441658 ISSN: 1432-0614
- PFEFFER ET AL: "Functional expression of lipase A from Candida antarctica in Escherichia coli-A prerequisite for high-throughput screening and directed evolution" JOURNAL OF MOLECULAR CATALYSIS. B, ENZYMATIC, ELSEVIER, AMSTERDAM,, NL, Bd. 45, Nr. 1-2, 16. Februar 2007 (2007-02-16), Seiten 62-67, XP005893194 ISSN: 1381-1177

### SEQUENZPROTOKOLL

<110> BASF AG
<120> Funktionale Expression von Triacylglycerol-Lipasen
<130> M/46349
<160> 13
<170> PatentIn version 3.3
<210> 1
   <211> 957
   <212> DNA
   <213> modifiziert aus Candida antarctica
<220>
   <221> misc_feature
   <222> (1)..(3)
   <223> erstes codierendes Basentriplett nach Startcodon
<220>
   <221> misc_feature
   <222> (952)..(954)
   <223> Stopcodon
<400> 1
   ctgccgagcg gtagcgatcc ggcgtttagc cagccgaaaa gcgttctgga tgcgggtctg      60
   acctgtcagg gtgcgagccc gagcagcgtt agcaaaccga ttctgctggt tccgggcacc     120
   ggcaccaccg gtccgcagag ctttgatagc aactggattc cgctgagcac ccagctgggt     180
   tataccccgt gttggattag cccgccgccg tttatgctga atgataccca ggtgaacacc     240
   gaatatatgg tgaacgcgat caccgcgctg tatgcgggta gcggcaataa taaactgccg     300
   gtgctgacct ggagccaggg tggtctggtt gcgcagtggg gtctgacctt ttttccgagc     360
   attcgcagca aagtggatcg tctgatggcg tttgcgccgg attataaagg caccgttctg     420
   gcgggtccgc tggatgcgct ggcggttagc gcgccgagcg tttggcagca gaccaccggt     480
   agcgcgctga ccaccgcgct gcgtaatgcg ggtggtctga cccagattgt tccgaccacc     540
   aatctgtata gcgcgaccga tgaaattgtt cagccgcagg ttagcaatag cccgctggat     600
   agcagctacc tgttcaacgg caaaaatgtt caggcgcagg cggtttgtgg tccgctgttt     660
   gtgattgatc atgcgggtag cctgaccagc cagtttagct atgtggttgg tcgtagcgcg     720
   ctgcgtagca ccaccggcca ggcgcgtagc gcggattatg gtatcaccga ttgcaatccg     780
   ctgccggcga atgatctgac cccggaacag aaagttgcgg cggcagcgct gctggcgccg     840
   gcagcggcgg ccattgttgc gggtccgaaa cagaattgtg aaccggatct gatgccgtat     900
   gcgcgtccgt ttgcggttgg taaacgtacc tgcagcggta ttgtgacccc gtaataa        957
<210> 2
   <211> 954
   <212> DNA
   <213> Candida antarctica
<220>
   <221> misc_feature
   <222> (1)..(3)
   <223> erstes codierendes Basentriplett nach Startcodon
<220>
   <221> misc_feature
   <222> (952)..(954)
   <223> Stopcodon
<400> 2
   ctaccttccg gttcggaccc tgccttttcg cagcccaagt cggtgctcga tgcgggtctg      60
   acctgccagg gtgcttcgcc atcctcggtc tccaaaccca tccttctcgt ccccggaacc     120
   ggcaccacag gtccacagtc gttcgactcg aactggatcc ccctctctgc gcagctgggt     180
   tacacaccct gctggatctc acccccgccg ttcatgctca acgacaccca ggtcaacacg     240
   gagtacatgg tcaacgccat caccacgctc tacgctggtt cgggcaacaa caagcttccc     300
   gtgctcacct ggtcccaggg tggtctggtt gcacagtggg gtctgacctt cttccccagt     360
   atcaggtcca aggtcgatcg acttatggcc tttgcgcccg actacaaggg caccgtcctc     420
   gccggccctc tcgatgcact cgcggttagt gcaccctccg tatggcagca aaccaccggt     480
   tcggcactca ctaccgcact ccgaaacgca ggtggtctga cccagatcgt gcccaccacc     540
   aacctctact cggcgaccga cgagatcgtt cagcctcagg tgtccaactc gccactcgac     600
   tcatcctacc tcttcaacgg aaagaacgtc caggcacagg ctgtgtgtgg gccgctgttc     660
   gtcatcgacc atgcaggctc gctcacctcg cagttctcct acgtcgtcgg tcgatccgcc     720
   ctgcgctcca ccacgggcca ggctcgtagt gcagactatg gcattacgga ctgcaaccct    780
   cttcccgcca atgatctgac tcccgagcaa aaggtcgccg cggctgcgct cctggcgccg     840
   gcggctgcag ccatcgtggc gggtccaaag cagaactgcg agcccgacct catgccctac     900
   gcccgcccct ttgcagtagg caaaaggacc tgctccggca tcgtcacccc ctga           954
<210> 3
   <211> 17
   <212> DNA
   <213> Artificial
<220>
   <223> aus pET32b+ abgeleitet
<400> 3
   taatacgact cactata 17
<210> 4
   <211> 67
   <212> DNA
   <213> Artificial
<220>
   <223> aus cspA (E.coli) abgeleitet
<400> 4
   ccgattaatc ataaatatga aaaataattg ttgcatcacc cgccaatgcg tggcttaatg      60
   cacatca                                                                67
<210> 5
   <211> 3383
   <212> DNA
   <213> Artificial
<220>
   <223> in pUC18 kloniertes calB_syn
<220>
   <221> misc_feature
   <222> (222)..(236)
   <223> von lacZ abgeleitetes Startcodon und codierende Sequenz
<220>
   <221> misc_feature
   <222> (237)..(1190)
   <223> calB_syn codierende Sequenz
<400> 5
   gcgcccaata cgcaaaccgc ctctccccgc gcgttggccg attcattaat gcagctggca      60
   cgacaggttt cccgactgga aagcgggcag tgagcgcaac gcaattaatg tgagttagct     120
   cactcattag gcaccccagg ctttacactt tatgcttccg gctcgtatgt tgtgtggaat     180
   tgtgagcgga taacaatttc acacaggaaa cagctatgac catgattacg aattcgctgc     240
   cgagcggtag cgatccggcg tttagccagc cgaaaagcgt tctggatgcg ggtctgacct     300
   gtcagggtgc gagcccgagc agcgttagca aaccgattct gctggttccg ggcaccggca     360
   ccaccggtcc gcagagcttt gatagcaact ggattccgct gagcacccag ctgggttata     420
   ccccgtgttg gattagcccg ccgccgttta tgctgaatga tacccaggtg aacaccgaat     480
   atatggtgaa cgcgatcacc gcgctgtatg cgggtagcgg caataataaa ctgccggtgc     540
   tgacctggag ccagggtggt ctggttgcgc agtggggtct gacctttttt ccgagcattc     600
   gcagcaaagt ggatcgtctg atggcgtttg cgccggatta taaaggcacc gttctggcgg     660
   gtccgctgga tgcgctggcg gttagcgcgc cgagcgtttg gcagcagacc accggtagcg     720
   cgctgaccac cgcgctgcgt aatgcgggtg gtctgaccca gattgttccg accaccaatc     780
   tgtatagcgc gaccgatgaa attgttcagc cgcaggttag caatagcccg ctggatagca     840
   gctacctgtt caacggcaaa aatgttcagg cgcaggcggt ttgtggtccg ctgtttgtga     900
   ttgatcatgc gggtagcctg accagccagt ttagctatgt ggttggtcgt agcgcgctgc     960
   gtagcaccac cggccaggcg cgtagcgcgg attatggtat caccgattgc aatccgctgc    1020
   cggcgaatga tctgaccccg gaacagaaag ttgcggcggc agcgctgctg gcgccggcag    1080
   cggcggccat tgttgcgggt ccgaaacaga attgtgaacc ggatctgatg ccgtatgcgc    1140
   gtccgtttgc ggttggtaaa cgtacctgca gcggtattgt gaccccgtaa taacatatgg    1200
   tgcactctca gtacaatctg ctctgatgcc gcatagttaa gccagccccg acacccgcca    1260
   acacccgctg acgcgccctg acgggcttgt ctgctcccgg catccgctta cagacaagct 1320
   gtgaccgtct ccgggagctg catgtgtcag aggttttcac cgtcatcacc gaaacgcgcg 1380
   agacgaaagg gcctcgtgat acgcctattt ttataggtta atgtcatgat aataatggtt 1440
   tcttagacgt caggtggcac ttttcgggga aatgtgcgcg gaacccctat ttgtttattt 1500
   ttctaaatac attcaaatat gtatccgctc atgagacaat aaccctgata aatgcttcaa 1560
   taatattgaa aaaggaagag tatgagtatt caacatttcc gtgtcgccct tattcccttt 1620
   tttgcggcat tttgccttcc tgtttttgct cacccagaaa cgctggtgaa agtaaaagat 1680
   gctgaagatc agttgggtgc acgagtgggt tacatcgaac tggatctcaa cagcggtaag 1740
   atccttgaga gttttcgccc cgaagaacgt tttccaatga tgagcacttt taaagttctg 1800
   ctatgtggcg cggtattatc ccgtattgac gccgggcaag agcaactcgg tcgccgcata 1860
   cactattctc agaatgactt ggttgagtac tcaccagtca cagaaaagca tcttacggat 1920
   ggcatgacag taagagaatt atgcagtgct gccataacca tgagtgataa cactgcggcc 1980
   aacttacttc tgacaacgat cggaggaccg aaggagctaa ccgctttttt gcacaacatg 2040
   ggggatcatg taactcgcct tgatcgttgg gaaccggagc tgaatgaagc cataccaaac 2100
   gacgagcgtg acaccacgat gcctgtagca atggcaacaa cgttgcgcaa actattaact 2160
   ggcgaactac ttactctagc ttcccggcaa caattaatag actggatgga ggcggataaa 2220
   gttgcaggac cacttctgcg ctcggccctt ccggctggct ggtttattgc tgataaatct 2280
   ggagccggtg agcgtgggtc tcgcggtatc attgcagcac tggggccaga tggtaagccc 2340
   tcccgtatcg tagttatcta cacgacgggg agtcaggcaa ctatggatga acgaaataga 2400
   cagatcgctg agataggtgc ctcactgatt aagcattggt aactgtcaga ccaagtttac 2460
   tcatatatac tttagattga tttaaaactt catttttaat ttaaaaggat ctaggtgaag 2520
   atcctttttg ataatctcat gaccaaaatc ccttaacgtg agttttcgtt ccactgagcg 2580
   tcagaccccg tagaaaagat caaaggatct tcttgagatc ctttttttct gcgcgtaatc 2640
   tgctgcttgc aaacaaaaaa accaccgcta ccagcggtgg tttgtttgcc ggatcaagag 2700
   ctaccaactc tttttccgaa ggtaactggc ttcagcagag cgcagatacc aaatactgtc 2760
   cttctagtgt agccgtagtt aggccaccac ttcaagaact ctgtagcacc gcctacatac 2820
   ctcgctctgc taatcctgtt accagtggct gctgccagtg gcgataagtc gtgtcttacc 2880
   gggttggact caagacgata gttaccggat aaggcgcagc ggtcgggctg aacggggggt 2940
   tcgtgcacac agcccagctt ggagcgaacg acctacaccg aactgagata cctacagcgt 3000
   gagctatgag aaagcgccac gcttcccgaa gggagaaagg cggacaggta tccggtaagc 3060
   ggcagggtcg gaacaggaga gcgcacgagg gagcttccag ggggaaacgc ctggtatctt 3120
   tatagtcctg tcgggtttcg ccacctctga cttgagcgtc gatttttgtg atgctcgtca 3180
   ggggggcgga gcctatggaa aaacgccagc aacgcggcct ttttacggtt cctggccttt 3240
   tgctggcctt ttgctcacat gttctttcct gcgttatccc ctgattctgt ggataaccgt 3300
   attaccgcct ttgagtgagc tgataccgct cgccgcagcc gaacgaccga gcgcagcgag    3360
   tcagtgagcg aggaagcgga aga                                            3383
<210> 6
   <211> 3333
   <212> DNA
   <213> Artificial
<220>
   <223> in pUC18 kloniertes calB_wt
<220>
   <221> misc_feature
   <222> (222)..(236)
   <223> von lacZ abgeleitetes Startcodon und codierende Sequenz
<220>
   <221> misc_feature
   <222> (237)..(1190)
   <223> calB_wt codierende Sequenz
<400> 6
   gcgcccaata cgcaaaccgc ctctccccgc gcgttggccg attcattaat gcagctggca     60
   cgacaggttt cccgactgga aagcgggcag tgagcgcaac gcaattaatg tgagttagct    120
   cactcattag gcaccccagg ctttacactt tatgcttccg gctcgtatgt tgtgtggaat    180
   tgtgagcgga taacaatttc acacaggaaa cagctatgac catgattacg aattcgctac    240
   cttccggttc ggaccctgcc ttttcgcagc ccaagtcggt gctcgatgcg ggtctgacct    300
   gccagggtgc ttcgccatcc tcggtctcca aacccatcct tctcgtcccc ggaaccggca    360
   ccacaggtcc acagtcgttc gactcgaact ggatccccct ctctgcgcag ctgggttaca    420
   caccctgctg gatctcaccc ccgccgttca tgctcaacga cacccaggtc aacacggagt    480
   acatggtcaa cgccatcacc acgctctacg ctggttcggg caacaacaag cttcccgtgc    540
   tcacctggtc ccagggtggt ctggttgcac agtggggtct gaccttcttc cccagtatca    600
   ggtccaaggt cgatcgactt atggcctttg cgcccgacta caagggcacc gtcctcgccg    660
   gccctctcga tgcactcgcg gttagtgcac cctccgtatg gcagcaaacc accggttcgg    720
   cactcactac cgcactccga aacgcaggtg gtctgaccca gatcgtgccc accaccaacc    780
   tctactcggc gaccgacgag atcgttcagc ctcaggtgtc caactcgcca ctcgactcat    840
   cctacctctt caacggaaag aacgtccagg cacaggctgt gtgtgggccg ctgttcgtca    900
   tcgaccatgc aggctcgctc acctcgcagt tctcctacgt cgtcggtcga tccgccctgc    960
   gctccaccac gggccaggct cgtagtgcag actatggcat tacggactgc aaccctcttc   1020
   ccgccaatga tctgactccc gagcaaaagg tcgccgcggc tgcgctcctg gcgccggcgg   1080
   ctgcagccat cgtggcgggt ccaaagcaga actgcgagcc cgacctcatg ccctacgccc   1140
   gcccctttgc agtaggcaaa aggacctgct ccggcatcgt caccccctaa gccagccccg   1200
   acacccgcca acacccgctg acgcgccctg acgggcttgt ctgctcccgg catccgctta   1260
   cagacaagct gtgaccgtct ccgggagctg catgtgtcag aggttttcac cgtcatcacc 1320
   gaaacgcgcg agacgaaagg gcctcgtgat acgcctattt ttataggtta atgtcatgat 1380
   aataatggtt tcttagacgt caggtggcac ttttcgggga aatgtgcgcg gaacccctat 1440
   ttgtttattt ttctaaatac attcaaatat gtatccgctc atgagacaat aaccctgata 1500
   aatgcttcaa taatattgaa aaaggaagag tatgagtatt caacatttcc gtgtcgccct 1560
   tattcccttt tttgcggcat tttgccttcc tgtttttgct cacccagaaa cgctggtgaa 1620
   agtaaaagat gctgaagatc agttgggtgc acgagtgggt tacatcgaac tggatctcaa 1680
   cagcggtaag atccttgaga gttttcgccc cgaagaacgt tttccaatga tgagcacttt 1740
   taaagttctg ctatgtggcg cggtattatc ccgtattgac gccgggcaag agcaactcgg 1800
   tcgccgcata cactattctc agaatgactt ggttgagtac tcaccagtca cagaaaagca 1860
   tcttacggat ggcatgacag taagagaatt atgcagtgct gccataacca tgagtgataa 1920
   cactgcggcc aacttacttc tgacaacgat cggaggaccg aaggagctaa ccgctttttt 1980
   gcacaacatg ggggatcatg taactcgcct tgatcgttgg gaaccggagc tgaatgaagc 2040
   cataccaaac gacgagcgtg acaccacgat gcctgtagca atggcaacaa cgttgcgcaa 2100
   actattaact ggcgaactac ttactctagc ttcccggcaa caattaatag actggatgga 2160
   ggcggataaa gttgcaggac cacttctgcg ctcggccctt ccggctggct ggtttattgc 2220
   tgataaatct ggagccggtg agcgtgggtc tcgcggtatc attgcagcac tggggccaga 2280
   tggtaagccc tcccgtatcg tagttatcta cacgacgggg agtcaggcaa ctatggatga 2340
   acgaaataga cagatcgctg agataggtgc ctcactgatt aagcattggt aactgtcaga 2400
   ccaagtttac tcatatatac tttagattga tttaaaactt catttttaat ttaaaaggat 2460
   ctaggtgaag atcctttttg ataatctcat gaccaaaatc ccttaacgtg agttttcgtt 2520
   ccactgagcg tcagaccccg tagaaaagat caaaggatct tcttgagatc ctttttttct 2580
   gcgcgtaatc tgctgcttgc aaacaaaaaa accaccgcta ccagcggtgg tttgtttgcc 2640
   ggatcaagag ctaccaactc tttttccgaa ggtaactggc ttcagcagag cgcagatacc 2700
   aaatactgtc cttctagtgt agccgtagtt aggccaccac ttcaagaact ctgtagcacc 2760
   gcctacatac ctcgctctgc taatcctgtt accagtggct gctgccagtg gcgataagtc 2820
   gtgtcttacc gggttggact caagacgata gttaccggat aaggcgcagc ggtcgggctg 2880
   aacggggggt tcgtgcacac agcccagctt ggagcgaacg acctacaccg aactgagata 2940
   cctacagcgt gagctatgag aaagcgccac gcttcccgaa gggagaaagg cggacaggta 3000
   tccggtaagc ggcagggtcg gaacaggaga gcgcacgagg gagcttccag ggggaaacgc 3060
   ctggtatctt tatagtcctg tcgggtttcg ccacctctga cttgagcgtc gatttttgtg 3120
   atgctcgtca ggggggcgga gcctatggaa aaacgccagc aacgcggcct ttttacggtt 3180
   cctggccttt tgctggcctt ttgctcacat gttctttcct gcgttatccc ctgattctgt 3240
   ggataaccgt attaccgcct ttgagtgagc tgataccgct cgccgcagcc gaacgaccga 3300
   gcgcagcgag tcagtgagcg aggaagcgga aga 3333
<210> 7
   <211> 6835
   <212> DNA
   <213> Artificial
<220>
   <223> in pET32b+ kloniertes calB_syn
<220>
   <221> misc_feature
   <222> (1)..(501)
   <223> Trx-Tag, dessen ATG auch als Startcodon fungiert
<220>
   <221> misc_feature
   <222> (502)..(1455)
   <223> calB_syn codierende Sequenz
<400> 7
   atgagcgata aaattattca cctgactgac gacagttttg acacggatgt actcaaagcg      60
   gacggggcga tcctcgtcga tttctgggca gagtggtgcg gtccgtgcaa aatgatcgcc     120
   ccgattctgg atgaaatcgc tgacgaatat cagggcaaac tgaccgttgc aaaactgaac     180
   atcgatcaaa accctggcac tgcgccgaaa tatggcatcc gtggtatccc gactctgctg     240
   ctgttcaaaa acggtgaagt ggcggcaacc aaagtgggtg cactgtctaa aggtcagttg     300
   aaagagttcc tcgacgctaa cctggccggt tctggttctg gccatatgca ccatcatcat     360
   catcattctt ctggtctggt gccacgcggt tctggtatga aagaaaccgc tgctgctaaa     420
   ttcgaacgcc agcacatgga cagcccagat ctgggtaccg acgacgacga caaggccatg     480
   gcgatatcgg atccgaattc gctgccgagc ggtagcgatc cggcgtttag ccagccgaaa     540
   agcgttctgg atgcgggtct gacctgtcag ggtgcgagcc cgagcagcgt tagcaaaccg     600
   attctgctgg ttccgggcac cggcaccacc ggtccgcaga gctttgatag caactggatt     660
   ccgctgagca cccagctggg ttataccccg tgttggatta gcccgccgcc gtttatgctg     720
   aatgataccc aggtgaacac cgaatatatg gtgaacgcga tcaccgcgct gtatgcgggt     780
   agcggcaata ataaactgcc ggtgctgacc tggagccagg gtggtctggt tgcgcagtgg     840
   ggtctgacct tttttccgag cattcgcagc aaagtggatc gtctgatggc gtttgcgccg     900
   gattataaag gcaccgttct ggcgggtccg ctggatgcgc tggcggttag cgcgccgagc     960
   gtttggcagc agaccaccgg tagcgcgctg accaccgcgc tgcgtaatgc gggtggtctg    1020
   acccagattg ttccgaccac caatctgtat agcgcgaccg atgaaattgt tcagccgcag    1080
   gttagcaata gcccgctgga tagcagctac ctgttcaacg gcaaaaatgt tcaggcgcag    1140
   gcggtttgtg gtccgctgtt tgtgattgat catgcgggta gcctgaccag ccagtttagc    1200
   tatgtggttg gtcgtagcgc gctgcgtagc accaccggcc aggcgcgtag cgcggattat    1260
   ggtatcaccg attgcaatcc gctgccggcg aatgatctga ccccggaaca gaaagttgcg 1320
   gcggcagcgc tgctggcgcc ggcagcggcg gccattgttg cgggtccgaa acagaattgt 1380
   gaaccggatc tgatgccgta tgcgcgtccg tttgcggttg gtaaacgtac ctgcagcggt 1440
   attgtgaccc cgtaagcggc cgcactcgag caccaccacc accaccactg agatccggct 1500
   gctaacaaag cccgaaagga agctgagttg gctgctgcca ccgctgagca ataactagca 1560
   taaccccttg gggcctctaa acgggtcttg aggggttttt tgctgaaagg aggaactata 1620
   tccggattgg cgaatgggac gcgccctgta gcggcgcatt aagcgcggcg ggtgtggtgg 1680
   ttacgcgcag cgtgaccgct acacttgcca gcgccctagc gcccgctcct ttcgctttct 1740
   tcccttcctt tctcgccacg ttcgccggct ttccccgtca agctctaaat cgggggctcc 1800
   ctttagggtt ccgatttagt gctttacggc acctcgaccc caaaaaactt gattagggtg 1860
   atggttcacg tagtgggcca tcgccctgat agacggtttt tcgccctttg acgttggagt 1920
   ccacgttctt taatagtgga ctcttgttcc aaactggaac aacactcaac cctatctcgg 1980
   tctattcttt tgatttataa gggattttgc cgatttcggc ctattggtta aaaaatgagc 2040
   tgatttaaca aaaatttaac gcgaatttta acaaaatatt aacgtttaca atttcaggtg 2100
   gcacttttcg gggaaatgtg cgcggaaccc ctatttgttt atttttctaa atacattcaa 2160
   atatgtatcc gctcatgaga caataaccct gataaatgct tcaataatat tgaaaaagga 2220
   agagtatgag tattcaacat ttccgtgtcg cccttattcc cttttttgcg gcattttgcc 2280
   ttcctgtttt tgctcaccca gaaacgctgg tgaaagtaaa agatgctgaa gatcagttgg 2340
   gtgcacgagt gggttacatc gaactggatc tcaacagcgg taagatcctt gagagttttc 2400
   gccccgaaga acgttttcca atgatgagca cttttaaagt tctgctatgt ggcgcggtat 2460
   tatcccgtat tgacgccggg caagagcaac tcggtcgccg catacactat tctcagaatg 2520
   acttggttga gtactcacca gtcacagaaa agcatcttac ggatggcatg acagtaagag 2580
   aattatgcag tgctgccata accatgagtg ataacactgc ggccaactta cttctgacaa 2640
   cgatcggagg accgaaggag ctaaccgctt ttttgcacaa catgggggat catgtaactc 2700
   gccttgatcg ttgggaaccg gagctgaatg aagccatacc aaacgacgag cgtgacacca 2760
   cgatgcctgc agcaatggca acaacgttgc gcaaactatt aactggcgaa ctacttactc 2820
   tagcttcccg gcaacaatta atagactgga tggaggcgga taaagttgca ggaccacttc 2880
   tgcgctcggc ccttccggct ggctggttta ttgctgataa atctggagcc ggtgagcgtg 2940
   ggtctcgcgg tatcattgca gcactggggc cagatggtaa gccctcccgt atcgtagtta 3000
   tctacacgac ggggagtcag gcaactatgg atgaacgaaa tagacagatc gctgagatag 3060
   gtgcctcact gattaagcat tggtaactgt cagaccaagt ttactcatat atactttaga 3120
   ttgatttaaa acttcatttt taatttaaaa ggatctaggt gaagatcctt tttgataatc 3180
   tcatgaccaa aatcccttaa cgtgagtttt cgttccactg agcgtcagac cccgtagaaa 3240
   agatcaaagg atcttcttga gatccttttt ttctgcgcgt aatctgctgc ttgcaaacaa 3300
   aaaaaccacc gctaccagcg gtggtttgtt tgccggatca agagctacca actctttttc 3360
   cgaaggtaac tggcttcagc agagcgcaga taccaaatac tgtccttcta gtgtagccgt 3420
   agttaggcca ccacttcaag aactctgtag caccgcctac atacctcgct ctgctaatcc 3480
   tgttaccagt ggctgctgcc agtggcgata agtcgtgtct taccgggttg gactcaagac 3540
   gatagttacc ggataaggcg cagcggtcgg gctgaacggg gggttcgtgc acacagccca 3600
   gcttggagcg aacgacctac accgaactga gatacctaca gcgtgagcta tgagaaagcg 3660
   ccacgcttcc cgaagggaga aaggcggaca ggtatccggt aagcggcagg gtcggaacag 3720
   gagagcgcac gagggagctt ccagggggaa acgcctggta tctttatagt cctgtcgggt 3780
   ttcgccacct ctgacttgag cgtcgatttt tgtgatgctc gtcagggggg cggagcctat 3840
   ggaaaaacgc cagcaacgcg gcctttttac ggttcctggc cttttgctgg ccttttgctc 3900
   acatgttctt tcctgcgtta tcccctgatt ctgtggataa ccgtattacc gcctttgagt 3960
   gagctgatac cgctcgccgc agccgaacga ccgagcgcag cgagtcagtg agcgaggaag 4020
   cggaagagcg cctgatgcgg tattttctcc ttacgcatct gtgcggtatt tcacaccgca 4080
   tatatggtgc actctcagta caatctgctc tgatgccgca tagttaagcc agtatacact 4140
   ccgctatcgc tacgtgactg ggtcatggct gcgccccgac acccgccaac acccgctgac 4200
   gcgccctgac gggcttgtct gctcccggca tccgcttaca gacaagctgt gaccgtctcc 4260
   gggagctgca tgtgtcagag gttttcaccg tcatcaccga aacgcgcgag gcagctgcgg 4320
   taaagctcat cagcgtggtc gtgaagcgat tcacagatgt ctgcctgttc atccgcgtcc 4380
   agctcgttga gtttctccag aagcgttaat gtctggcttc tgataaagcg ggccatgtta 4440
   agggcggttt tttcctgttt ggtcactgat gcctccgtgt aagggggatt tctgttcatg 4500
   ggggtaatga taccgatgaa acgagagagg atgctcacga tacgggttac tgatgatgaa 4560
   catgcccggt tactggaacg ttgtgagggt aaacaactgg cggtatggat gcggcgggac 4620
   cagagaaaaa tcactcaggg tcaatgccag cgcttcgtta atacagatgt aggtgttcca 4680
   cagggtagcc agcagcatcc tgcgatgcag atccggaaca taatggtgca gggcgctgac 4740
   ttccgcgttt ccagacttta cgaaacacgg aaaccgaaga ccattcatgt tgttgctcag 4800
   gtcgcagacg ttttgcagca gcagtcgctt cacgttcgct cgcgtatcgg tgattcattc 4860
   tgctaaccag taaggcaacc ccgccagcct agccgggtcc tcaacgacag gagcacgatc 4920
   atgcgcaccc gtggggccgc catgccggcg ataatggcct gcttctcgcc gaaacgtttg 4980
   gtggcgggac cagtgacgaa ggcttgagcg agggcgtgca agattccgaa taccgcaagc 5040
   gacaggccga tcatcgtcgc gctccagcga aagcggtcct cgccgaaaat gacccagagc 5100
   gctgccggca cctgtcctac gagttgcatg ataaagaaga cagtcataag tgcggcgacg 5160
   atagtcatgc cccgcgccca ccggaaggag ctgactgggt tgaaggctct caagggcatc 5220
   ggtcgagatc ccggtgccta atgagtgagc taacttacat taattgcgtt gcgctcactg 5280
   cccgctttcc agtcgggaaa cctgtcgtgc cagctgcatt aatgaatcgg ccaacgcgcg   5340
   gggagaggcg gtttgcgtat tgggcgccag ggtggttttt cttttcacca gtgagacggg   5400
   caacagctga ttgcccttca ccgcctggcc ctgagagagt tgcagcaagc ggtccacgct   5460
   ggtttgcccc agcaggcgaa aatcctgttt gatggtggtt aacggcggga tataacatga   5520
   gctgtcttcg gtatcgtcgt atcccactac cgagatgtcc gcaccaacgc gcagcccgga   5580
   ctcggtaatg gcgcgcattg cgcccagcgc catctgatcg ttggcaacca gcatcgcagt   5640
   gggaacgatg ccctcattca gcatttgcat ggtttgttga aaaccggaca tggcactcca   5700
   gtcgccttcc cgttccgcta tcggctgaat ttgattgcga gtgagatatt tatgccagcc   5760
   agccagacgc agacgcgccg agacagaact taatgggccc gctaacagcg cgatttgctg   5820
   gtgacccaat gcgaccagat gctccacgcc cagtcgcgta ccgtcttcat gggagaaaat   5880
   aatactgttg atgggtgtct ggtcagagac atcaagaaat aacgccggaa cattagtgca   5940
   ggcagcttcc acagcaatgg catcctggtc atccagcgga tagttaatga tcagcccact   6000
   gacgcgttgc gcgagaagat tgtgcaccgc cgctttacag gcttcgacgc cgcttcgttc   6060
   taccatcgac accaccacgc tggcacccag ttgatcggcg cgagatttaa tcgccgcgac   6120
   aatttgcgac ggcgcgtgca gggccagact ggaggtggca acgccaatca gcaacgactg   6180
   tttgcccgcc agttgttgtg ccacgcggtt gggaatgtaa ttcagctccg ccatcgccgc   6240
   ttccactttt tcccgcgttt tcgcagaaac gtggctggcc tggttcacca cgcgggaaac   6300
   ggtctgataa gagacaccgg catactctgc gacatcgtat aacgttactg gtttcacatt   6360
   caccaccctg aattgactct cttccgggcg ctatcatgcc ataccgcgaa aggttttgcg   6420
   ccattcgatg gtgtccggga tctcgacgct ctcccttatg cgactcctgc attaggaagc   6480
   agcccagtag taggttgagg ccgttgagca ccgccgccgc aaggaatggt gcatgcaagg   6540
   agatggcgcc caacagtccc ccggccacgg ggcctgccac catacccacg ccgaaacaag   6600
   cgctcatgag cccgaagtgg cgagcccgat cttccccatc ggtgatgtcg gcgatatagg   6660
   cgccagcaac cgcacctgtg gcgccggtga tgccggccac gatgcgtccg gcgtagagga   6720
   tcgagatcga tctcgatccc gcgaaattaa tacgactcac tataggggaa ttgtgagcgg   6780
   ataacaattc ccctctagaa ataattttgt ttaactttaa gaaggagata tacat        6835
<210> 8
   <211> 6835
   <212> DNA
   <213> Artificial
<220>
   <223> in pET32b+ kloniertes calB_wt
<220>
   <221> misc_feature
   <222> (1)..(501)
   <223> Trx-Tag, dessen ATG auch als Startcodon fungiert
<220>
   <221> misc_feature
   <222> (502)..(1455)
   <223> calB_wt codierende Sequenz
<400> 8
   atgagcgata aaattattca cctgactgac gacagttttg acacggatgt actcaaagcg 60
   gacggggcga tcctcgtcga tttctgggca gagtggtgcg gtccgtgcaa aatgatcgcc 120
   ccgattctgg atgaaatcgc tgacgaatat cagggcaaac tgaccgttgc aaaactgaac 180
   atcgatcaaa accctggcac tgcgccgaaa tatggcatcc gtggtatccc gactctgctg 240
   ctgttcaaaa acggtgaagt ggcggcaacc aaagtgggtg cactgtctaa aggtcagttg 300
   aaagagttcc tcgacgctaa cctggccggt tctggttctg gccatatgca ccatcatcat 360
   catcattctt ctggtctggt gccacgcggt tctggtatga aagaaaccgc tgctgctaaa 420
   ttcgaacgcc agcacatgga cagcccagat ctgggtaccg acgacgacga caaggccatg 480
   gcgatatcgg atccgaattc gctaccttcc ggttcggacc ctgccttttc gcagcccaag 540
   tcggtgctcg atgcgggtct gacctgccag ggtgcttcgc catcctcggt ctccaaaccc 600
   atccttctcg tccccggaac cggcaccaca ggtccacagt cgttcgactc gaactggatc 660
   cccctctctg cgcagctggg ttacacaccc tgctggatct cacccccgcc gttcatgctc 720
   aacgacaccc aggtcaacac ggagtacatg gtcaacgcca tcaccacgct ctacgctggt 780
   tcgggcaaca acaagcttcc cgtgctcacc tggtcccagg gtggtctggt tgcacagtgg 840
   ggtctgacct tcttccccag tatcaggtcc aaggtcgatc gacttatggc ctttgcgccc 900
   gactacaagg gcaccgtcct cgccggccct ctcgatgcac tcgcggttag tgcaccctcc 960
   gtatggcagc aaaccaccgg ttcggcactc actaccgcac tccgaaacgc aggtggtctg 1020
   acccagatcg tgcccaccac caacctctac tcggcgaccg acgagatcgt tcagcctcag 1080
   gtgtccaact cgccactcga ctcatcctac ctcttcaacg gaaagaacgt ccaggcacag 1140
   gctgtgtgtg ggccgctgtt cgtcatcgac catgcaggct cgctcacctc gcagttctcc 1200
   tacgtcgtcg gtcgatccgc cctgcgctcc accacgggcc aggctcgtag tgcagactat 1260
   ggcattacgg actgcaaccc tcttcccgcc aatgatctga ctcccgagca aaaggtcgcc 1320
   gcggctgcgc tcctggcgcc ggcggctgca gccatcgtgg cgggtccaaa gcagaactgc 1380
   gagcccgacc tcatgcccta cgcccgcccc tttgcagtag gcaaaaggac ctgctccggc 1440
   atcgtcaccc cctgagcggc cgcactcgag caccaccacc accaccactg agatccggct 1500
   gctaacaaag cccgaaagga agctgagttg gctgctgcca ccgctgagca ataactagca 1560
   taaccccttg gggcctctaa acgggtcttg aggggttttt tgctgaaagg aggaactata 1620
   tccggattgg cgaatgggac gcgccctgta gcggcgcatt aagcgcggcg ggtgtggtgg 1680
   ttacgcgcag cgtgaccgct acacttgcca gcgccctagc gcccgctcct ttcgctttct 1740
   tcccttcctt tctcgccacg ttcgccggct ttccccgtca agctctaaat cgggggctcc 1800
   ctttagggtt ccgatttagt gctttacggc acctcgaccc caaaaaactt gattagggtg 1860
   atggttcacg tagtgggcca tcgccctgat agacggtttt tcgccctttg acgttggagt 1920
   ccacgttctt taatagtgga ctcttgttcc aaactggaac aacactcaac cctatctcgg 1980
   tctattcttt tgatttataa gggattttgc cgatttcggc ctattggtta aaaaatgagc 2040
   tgatttaaca aaaatttaac gcgaatttta acaaaatatt aacgtttaca atttcaggtg 2100
   gcacttttcg gggaaatgtg cgcggaaccc ctatttgttt atttttctaa atacattcaa 2160
   atatgtatcc gctcatgaga caataaccct gataaatgct tcaataatat tgaaaaagga 2220
   agagtatgag tattcaacat ttccgtgtcg cccttattcc cttttttgcg gcattttgcc 2280
   ttcctgtttt tgctcaccca gaaacgctgg tgaaagtaaa agatgctgaa gatcagttgg 2340
   gtgcacgagt gggttacatc gaactggatc tcaacagcgg taagatcctt gagagttttc 2400
   gccccgaaga acgttttcca atgatgagca cttttaaagt tctgctatgt ggcgcggtat 2460
   tatcccgtat tgacgccggg caagagcaac tcggtcgccg catacactat tctcagaatg 2520
   acttggttga gtactcacca gtcacagaaa agcatcttac ggatggcatg acagtaagag 2580
   aattatgcag tgctgccata accatgagtg ataacactgc ggccaactta cttctgacaa 2640
   cgatcggagg accgaaggag ctaaccgctt ttttgcacaa catgggggat catgtaactc 2700
   gccttgatcg ttgggaaccg gagctgaatg aagccatacc aaacgacgag cgtgacacca 2760
   cgatgcctgc agcaatggca acaacgttgc gcaaactatt aactggcgaa ctacttactc 2820
   tagcttcccg gcaacaatta atagactgga tggaggcgga taaagttgca ggaccacttc 2880
   tgcgctcggc ccttccggct ggctggttta ttgctgataa atctggagcc ggtgagcgtg 2940
   ggtctcgcgg tatcattgca gcactggggc cagatggtaa gccctcccgt atcgtagtta 3000
   tctacacgac ggggagtcag gcaactatgg atgaacgaaa tagacagatc gctgagatag 3060
   gtgcctcact gattaagcat tggtaactgt cagaccaagt ttactcatat atactttaga 3120
   ttgatttaaa acttcatttt taatttaaaa ggatctaggt gaagatcctt tttgataatc 3180
   tcatgaccaa aatcccttaa cgtgagtttt cgttccactg agcgtcagac cccgtagaaa 3240
   agatcaaagg atcttcttga gatccttttt ttctgcgcgt aatctgctgc ttgcaaacaa 3300
   aaaaaccacc gctaccagcg gtggtttgtt tgccggatca agagctacca actctttttc 3360
   cgaaggtaac tggcttcagc agagcgcaga taccaaatac tgtccttcta gtgtagccgt 3420
   agttaggcca ccacttcaag aactctgtag caccgcctac atacctcgct ctgctaatcc 3480
   tgttaccagt ggctgctgcc agtggcgata agtcgtgtct taccgggttg gactcaagac 3540
   gatagttacc ggataaggcg cagcggtcgg gctgaacggg gggttcgtgc acacagccca 3600
   gcttggagcg aacgacctac accgaactga gatacctaca gcgtgagcta tgagaaagcg 3660
   ccacgcttcc cgaagggaga aaggcggaca ggtatccggt aagcggcagg gtcggaacag 3720
   gagagcgcac gagggagctt ccagggggaa acgcctggta tctttatagt cctgtcgggt 3780
   ttcgccacct ctgacttgag cgtcgatttt tgtgatgctc gtcagggggg cggagcctat 3840
   ggaaaaacgc cagcaacgcg gcctttttac ggttcctggc cttttgctgg ccttttgctc 3900
   acatgttctt tcctgcgtta tcccctgatt ctgtggataa ccgtattacc gcctttgagt 3960
   gagctgatac cgctcgccgc agccgaacga ccgagcgcag cgagtcagtg agcgaggaag 4020
   cggaagagcg cctgatgcgg tattttctcc ttacgcatct gtgcggtatt tcacaccgca 4080
   tatatggtgc actctcagta caatctgctc tgatgccgca tagttaagcc agtatacact 4140
   ccgctatcgc tacgtgactg ggtcatggct gcgccccgac acccgccaac acccgctgac 4200
   gcgccctgac gggcttgtct gctcccggca tccgcttaca gacaagctgt gaccgtctcc 4260
   gggagctgca tgtgtcagag gttttcaccg tcatcaccga aacgcgcgag gcagctgcgg 4320
   taaagctcat cagcgtggtc gtgaagcgat tcacagatgt ctgcctgttc atccgcgtcc 4380
   agctcgttga gtttctccag aagcgttaat gtctggcttc tgataaagcg ggccatgtta 4440
   agggcggttt tttcctgttt ggtcactgat gcctccgtgt aagggggatt tctgttcatg 4500
   ggggtaatga taccgatgaa acgagagagg atgctcacga tacgggttac tgatgatgaa 4560
   catgcccggt tactggaacg ttgtgagggt aaacaactgg cggtatggat gcggcgggac 4620
   cagagaaaaa tcactcaggg tcaatgccag cgcttcgtta atacagatgt aggtgttcca 4680
   cagggtagcc agcagcatcc tgcgatgcag atccggaaca taatggtgca gggcgctgac 4740
   ttccgcgttt ccagacttta cgaaacacgg aaaccgaaga ccattcatgt tgttgctcag 4800
   gtcgcagacg ttttgcagca gcagtcgctt cacgttcgct cgcgtatcgg tgattcattc 4860
   tgctaaccag taaggcaacc ccgccagcct agccgggtcc tcaacgacag gagcacgatc 4920
   atgcgcaccc gtggggccgc catgccggcg ataatggcct gcttctcgcc gaaacgtttg 4980
   gtggcgggac cagtgacgaa ggcttgagcg agggcgtgca agattccgaa taccgcaagc 5040
   gacaggccga tcatcgtcgc gctccagcga aagcggtcct cgccgaaaat gacccagagc 5100
   gctgccggca cctgtcctac gagttgcatg ataaagaaga cagtcataag tgcggcgacg 5160
   atagtcatgc cccgcgccca ccggaaggag ctgactgggt tgaaggctct caagggcatc 5220
   ggtcgagatc ccggtgccta atgagtgagc taacttacat taattgcgtt gcgctcactg 5280
   cccgctttcc agtcgggaaa cctgtcgtgc cagctgcatt aatgaatcgg ccaacgcgcg 5340
   gggagaggcg gtttgcgtat tgggcgccag ggtggttttt cttttcacca gtgagacggg 5400
   caacagctga ttgcccttca ccgcctggcc ctgagagagt tgcagcaagc ggtccacgct 5460
   ggtttgcccc agcaggcgaa aatcctgttt gatggtggtt aacggcggga tataacatga 5520
   gctgtcttcg gtatcgtcgt atcccactac cgagatgtcc gcaccaacgc gcagcccgga 5580
   ctcggtaatg gcgcgcattg cgcccagcgc catctgatcg ttggcaacca gcatcgcagt 5640
   gggaacgatg ccctcattca gcatttgcat ggtttgttga aaaccggaca tggcactcca 5700
   gtcgccttcc cgttccgcta tcggctgaat ttgattgcga gtgagatatt tatgccagcc 5760
   agccagacgc agacgcgccg agacagaact taatgggccc gctaacagcg cgatttgctg 5820
   gtgacccaat gcgaccagat gctccacgcc cagtcgcgta ccgtcttcat gggagaaaat 5880
   aatactgttg atgggtgtct ggtcagagac atcaagaaat aacgccggaa cattagtgca   5940
   ggcagcttcc acagcaatgg catcctggtc atccagcgga tagttaatga tcagcccact   6000
   gacgcgttgc gcgagaagat tgtgcaccgc cgctttacag gcttcgacgc cgcttcgttc   6060
   taccatcgac accaccacgc tggcacccag ttgatcggcg cgagatttaa tcgccgcgac   6120
   aatttgcgac ggcgcgtgca gggccagact ggaggtggca acgccaatca gcaacgactg   6180
   tttgcccgcc agttgttgtg ccacgcggtt gggaatgtaa ttcagctccg ccatcgccgc   6240
   ttccactttt tcccgcgttt tcgcagaaac gtggctggcc tggttcacca cgcgggaaac   6300
   ggtctgataa gagacaccgg catactctgc gacatcgtat aacgttactg gtttcacatt   6360
   caccaccctg aattgactct cttccgggcg ctatcatgcc ataccgcgaa aggttttgcg   6420
   ccattcgatg gtgtccggga tctcgacgct ctcccttatg cgactcctgc attaggaagc   6480
   agcccagtag taggttgagg ccgttgagca ccgccgccgc aaggaatggt gcatgcaagg   6540
   agatggcgcc caacagtccc ccggccacgg ggcctgccac catacccacg ccgaaacaag   6600
   cgctcatgag cccgaagtgg cgagcccgat cttccccatc ggtgatgtcg gcgatatagg   6660
   cgccagcaac cgcacctgtg gcgccggtga tgccggccac gatgcgtccg gcgtagagga   6720
   tcgagatcga tctcgatccc gcgaaattaa tacgactcac tataggggaa ttgtgagcgg   6780
   ataacaattc ccctctagaa ataattttgt ttaactttaa gaaggagata tacat        6835
<210> 9
   <211> 5307
   <212> DNA
   <213> Artificial
<220>
   <223> in pCOLDIII kloniertes calB_syn
<220>
   <221> misc_feature
   <222> (1)..(3)
   <223> Startcodon
<220>
   <221> misc_feature
   <222> (4)..(957)
   <223> calB_syn coderierende Sequenz
<400> 9
   atgctgccga gcggtagcga tccggcgttt agccagccga aaagcgttct ggatgcgggt      60
   ctgacctgtc agggtgcgag cccgagcagc gttagcaaac cgattctgct ggttccgggc     120
   accggcacca ccggtccgca gagctttgat agcaactgga ttccgctgag cacccagctg     180
   ggttataccc cgtgttggat tagcccgccg ccgtttatgc tgaatgatac ccaggtgaac     240
   accgaatata tggtgaacgc gatcaccgcg ctgtatgcgg gtagcggcaa taataaactg     300
   ccggtgctga cctggagcca gggtggtctg gttgcgcagt ggggtctgac cttttttccg    360
   agcattcgca gcaaagtgga tcgtctgatg gcgtttgcgc cggattataa aggcaccgtt     420
   ctggcgggtc cgctggatgc gctggcggtt agcgcgccga gcgtttggca gcagaccacc 480
   ggtagcgcgc tgaccaccgc gctgcgtaat gcgggtggtc tgacccagat tgttccgacc 540
   accaatctgt atagcgcgac cgatgaaatt gttcagccgc aggttagcaa tagcccgctg 600
   gatagcagct acctgttcaa cggcaaaaat gttcaggcgc aggcggtttg tggtccgctg 660
   tttgtgattg atcatgcggg tagcctgacc agccagttta gctatgtggt tggtcgtagc 720
   gcgctgcgta gcaccaccgg ccaggcgcgt agcgcggatt atggtatcac cgattgcaat 780
   ccgctgccgg cgaatgatct gaccccggaa cagaaagttg cggcggcagc gctgctggcg 840
   ccggcagcgg cggccattgt tgcgggtccg aaacagaatt gtgaaccgga tctgatgccg 900
   tatgcgcgtc cgtttgcggt tggtaaacgt acctgcagcg gtattgtgac cccgtaagaa 960
   ttcaagcttg tcgacctgca gtctagatag gtaatctctg cttaaaagca cagaatctaa 1020
   gatccctgcc atttggcggg gattttttta tttgttttca ggaaataaat aatcgatcgc 1080
   gtaataaaat ctattattat ttttgtgaag aataaatttg ggtgcaatga gaatgcgcag 1140
   gccctttcgt ctcgcgcgtt tcggtgatga cggtgaaaac ctctgacaca tgcagctccc 1200
   ggagacggtc acagcttgtc tgtaagcgga tgccgggagc agacaagccc gtcagggcgc 1260
   gtcagcgggt gttggcgggt gtcggggctg gcttaactat gcggcatcag agcagattgt 1320
   actgagagtg caccataaaa ttgtaaacgt taatattttg ttaaaattcg cgttaaattt 1380
   ttgttaaatc agctcatttt ttaaccaata ggccgaaatc ggcaaaatcc cttataaatc 1440
   aaaagaatag cccgagatag ggttgagtgt tgttccagtt tggaacaaga gtccactatt 1500
   aaagaacgtg gactccaacg tcaaagggcg aaaaaccgtc tatcagggcg atggcccact 1560
   acgtgaacca tcacccaaat caagtttttt ggggtcgagg tgccgtaaag cactaaatcg 1620
   gaaccctaaa gggagccccc gatttagagc ttgacgggga aagccggcga acgtggcgag 1680
   aaaggaaggg aagaaagcga aaggagcggg cgctagggcg ctggcaagtg tagcggtcac 1740
   gctgcgcgta accaccacac ccgccgcgct taatgcgccg ctacagggcg cgtactatgg 1800
   ttgctttgac gtatgcggtg tgaaataccg cacagatgcg taaggagaaa ataccgcatc 1860
   aggcgtcagg tggcactttt cggggaaatg tgcgcggaac ccctatttgt ttatttttct 1920
   aaatacattc aaatatgtat ccgctcatga gacaataacc ctgataaatg cttcaataat 1980
   attgaaaaag gaagagtatg agtattcaac atttccgtgt cgcccttatt cccttttttg 2040
   cggcattttg ccttcctgtt tttgctcacc cagaaacgct ggtgaaagta aaagatgctg 2100
   aagatcagtt gggtgcacga gtgggttaca tcgaactgga tctcaacagc ggtaagatcc 2160
   ttgagagttt tcgccccgaa gaacgttttc caatgatgag cacttttaaa gttctgctat 2220
   gtggcgcggt attatcccgt attgacgccg ggcaagagca actcggtcgc cgcatacact 2280
   attctcagaa tgacttggtt gagtactcac cagtcacaga aaagcatctt acggatggca 2340
   tgacagtaag agaattatgc agtgctgcca taaccatgag tgataacact gcggccaact 2400
   tacttctgac aacgatcgga ggaccgaagg agctaaccgc ttttttgcac aacatggggg 2460
   atcatgtaac tcgccttgat cgttgggaac cggagctgaa tgaagccata ccaaacgacg 2520
   agcgtgacac cacgatgcct gtagcaatgg caacaacgtt gcgcaaacta ttaactggcg 2580
   aactacttac tctagcttcc cggcaacaat taatagactg gatggaggcg gataaagttg 2640
   caggaccact tctgcgctcg gcccttccgg ctggctggtt tattgctgat aaatctggag 2700
   ccggtgagcg tgggtctcgc ggtatcattg cagcactggg gccagatggt aagccctccc 2760
   gtatcgtagt tatctacacg acggggagtc aggcaactat ggatgaacga aatagacaga 2820
   tcgctgagat aggtgcctca ctgattaagc attggtaact gtcagaccaa gtttactcat 2880
   atatacttta gattgattta aaacttcatt tttaatttaa aaggatctag gtgaagatcc 2940
   tttttgataa tctcatgacc aaaatccctt aacgtgagtt ttcgttccac tgagcgtcag 3000
   accccgtaga aaagatcaaa ggatcttctt gagatccttt ttttctgcgc gtaatctgct 3060
   gcttgcaaac aaaaaaacca ccgctaccag cggtggtttg tttgccggat caagagctac 3120
   caactctttt tccgaaggta actggcttca gcagagcgca gataccaaat actgttcttc 3180
   tagtgtagcc gtagttaggc caccacttca agaactctgt agcaccgcct acatacctcg 3240
   ctctgctaat cctgttacca gtggctgctg ccagtggcga taagtcgtgt cttaccgggt 3300
   tggactcaag acgatagtta ccggataagg cgcagcggtc gggctgaacg gggggttcgt 3360
   gcacacagcc cagcttggag cgaacgacct acaccgaact gagataccta cagcgtgagc 3420
   tatgagaaag cgccacgctt cccgaaggga gaaaggcgga caggtatccg gtaagcggca 3480
   gggtcggaac aggagagcgc acgagggagc ttccaggggg aaacgcctgg tatctttata 3540
   gtcctgtcgg gtttcgccac ctctgacttg agcgtcgatt tttgtgatgc tcgtcagggg 3600
   ggcggagcct atggaaaaac gccagcaacg cggccttttt acggttcctg gccttttgct 3660
   ggccttttgc tcacatagtc atgccccgcg cccaccggaa ggagctgact gggttgaagg 3720
   ctctcaaggg catcggtcga gatcccggtg cctaatgagt gagctaactt acattaattg 3780
   cgttgcgctc actgcccgct ttccagtcgg gaaacctgtc gtgccagctg cattaatgaa 3840
   tcggccaacg cgcggggaga ggcggtttgc gtattgggcg ccagggtggt ttttcttttc 3900
   accagtgaga cgggcaacag ctgattgccc ttcaccgcct ggccctgaga gagttgcagc 3960
   aagcggtcca cgctggtttg ccccagcagg cgaaaatcct gtttgatggt ggttaacggc 4020
   gggatataac atgagctgtc ttcggtatcg tcgtatccca ctaccgagat atccgcacca 4080
   acgcgcagcc cggactcggt aatggcgcgc attgcgccca gcgccatctg atcgttggca 4140
   accagcatcg cagtgggaac gatgccctca ttcagcattt gcatggtttg ttgaaaaccg 4200
   gacatggcac tccagtcgcc ttcccgttcc gctatcggct gaatttgatt gcgagtgaga 4260
   tatttatgcc agccagccag acgcagacgc gccgagacag aacttaatgg gcccgctaac 4320
   agcgcgattt gctggtgacc caatgcgacc agatgctcca cgcccagtcg cgtaccgtct 4380
   tcatgggaga aaataatact gttgatgggt gtctggtcag agacatcaag aaataacgcc    4440
   ggaacattag tgcaggcagc ttccacagca atggcatcct ggtcatccag cggatagtta    4500
   atgatcagcc cactgacgcg ttgcgcgaga agattgtgca ccgccgcttt acaggcttcg    4560
   acgccgcttc gttctaccat cgacaccacc acgctggcac ccagttgatc ggcgcgagat    4620
   ttaatcgccg cgacaatttg cgacggcgcg tgcagggcca gactggaggt ggcaacgcca    4680
   atcagcaacg actgtttgcc cgccagttgt tgtgccacgc ggttgggaat gtaattcagc    4740
   tccgccatcg ccgcttccac tttttcccgc gttttcgcag aaacgtggct ggcctggttc    4800
   accacgcggg aaacggtctg ataagagaca ccggcatact ctgcgacatc gtataacgtt    4860
   actggtttca cattcaccac cctgaattga ctctcttccg ggcgctatca tgccataccg    4920
   cgaaaggttt tgcgccattc gatggtgtcc gggatctcga cgctctccct tatgcgactc    4980
   ctgcattagg aagcagccca gtagtaggtt gaggccgttg agcaccgccg ccgcaaggaa    5040
   tggtgtggcc gattaatcat aaatatgaaa aataattgtt gcatcacccg ccaatgcgtg    5100
   gcttaatgca catcaaattg tgagcggata acaatttgat gtgctagcgc atatccagtg    5160
   tagtaaggca agtcccttca agagttatcg ttgatacccc tcgtagtgca cattccttta    5220
   acgcttcaaa atctgtaaag cacgccatat cgccgaaagg cacacttaat tattaagagg    5280
   taatacacca tgaatcacaa agtgcat                                        5307
<210> 10
   <211> 5307
   <212> DNA
   <213> Artificial
<220>
   <223> in pCOLDIII kloniertes calB_wt
<220>
   <221> misc_feature
   <222> (1)..(3)
   <223> Startcodon
<220>
   <221> misc_feature
   <222> (4)..(957)
   <223> calB_wt codierende Sequenz
<400> 10
   atgctacctt ccggttcgga ccctgccttt tcgcagccca agtcggtgct cgatgcgggt     60
   ctgacctgcc agggtgcttc gccatcctcg gtctccaaac ccatccttct cgtccccgga    120
   accggcacca caggtccaca gtcgttcgac tcgaactgga tccccctctc tgcgcagctg    180
   ggttacacac cctgctggat ctcacccccg ccgttcatgc tcaacgacac ccaggtcaac    240
   acggagtaca tggtcaacgc catcaccacg ctctacgctg gttcgggcaa caacaagctt    300
   cccgtgctca cctggtccca gggtggtctg gttgcacagt ggggtctgac cttcttcccc    360
   agtatcaggt ccaaggtcga tcgacttatg gcctttgcgc ccgactacaa gggcaccgtc    420
   ctcgccggcc ctctcgatgc actcgcggtt agtgcaccct ccgtatggca gcaaaccacc    480
   ggttcggcac tcactaccgc actccgaaac gcaggtggtc tgacccagat cgtgcccacc    540
   accaacctct actcggcgac cgacgagatc gttcagcctc aggtgtccaa ctcgccactc    600
   gactcatcct acctcttcaa cggaaagaac gtccaggcac aggctgtgtg tgggccgctg    660
   ttcgtcatcg accatgcagg ctcgctcacc tcgcagttct cctacgtcgt cggtcgatcc    720
   gccctgcgct ccaccacggg ccaggctcgt agtgcagact atggcattac ggactgcaac    780
   cctcttcccg ccaatgatct gactcccgag caaaaggtcg ccgcggctgc gctcctggcg    840
   ccggcggctg cagccatcgt ggcgggtcca aagcagaact gcgagcccga cctcatgccc    900
   tacgcccgcc cctttgcagt aggcaaaagg acctgctccg gcatcgtcac cccctgagaa    960
   ttcaagcttg tcgacctgca gtctagatag gtaatctctg cttaaaagca cagaatctaa   1020
   gatccctgcc atttggcggg gattttttta tttgttttca ggaaataaat aatcgatcgc   1080
   gtaataaaat ctattattat ttttgtgaag aataaatttg ggtgcaatga gaatgcgcag   1140
   gccctttcgt ctcgcgcgtt tcggtgatga cggtgaaaac ctctgacaca tgcagctccc   1200
   ggagacggtc acagcttgtc tgtaagcgga tgccgggagc agacaagccc gtcagggcgc   1260
   gtcagcgggt gttggcgggt gtcggggctg gcttaactat gcggcatcag agcagattgt   1320
   actgagagtg caccataaaa ttgtaaacgt taatattttg ttaaaattcg cgttaaattt   1380
   ttgttaaatc agctcatttt ttaaccaata ggccgaaatc ggcaaaatcc cttataaatc   1440
   aaaagaatag cccgagatag ggttgagtgt tgttccagtt tggaacaaga gtccactatt   1500
   aaagaacgtg gactccaacg tcaaagggcg aaaaaccgtc tatcagggcg atggcccact   1560
   acgtgaacca tcacccaaat caagtttttt ggggtcgagg tgccgtaaag cactaaatcg   1620
   gaaccctaaa gggagccccc gatttagagc ttgacgggga aagccggcga acgtggcgag   1680
   aaaggaaggg aagaaagcga aaggagcggg cgctagggcg ctggcaagtg tagcggtcac   1740
   gctgcgcgta accaccacac ccgccgcgct taatgcgccg ctacagggcg cgtactatgg   1800
   ttgctttgac gtatgcggtg tgaaataccg cacagatgcg taaggagaaa ataccgcatc   1860
   aggcgtcagg tggcactttt cggggaaatg tgcgcggaac ccctatttgt ttatttttct   1920
   aaatacattc aaatatgtat ccgctcatga gacaataacc ctgataaatg cttcaataat   1980
   attgaaaaag gaagagtatg agtattcaac atttccgtgt cgcccttatt cccttttttg    2040
   cggcattttg ccttcctgtt tttgctcacc cagaaacgct ggtgaaagta aaagatgctg   2100
   aagatcagtt gggtgcacga gtgggttaca tcgaactgga tctcaacagc ggtaagatcc   2160
   ttgagagttt tcgccccgaa gaacgttttc caatgatgag cacttttaaa gttctgctat   2220
   gtggcgcggt attatcccgt attgacgccg ggcaagagca actcggtcgc cgcatacact   2280
   attctcagaa tgacttggtt gagtactcac cagtcacaga aaagcatctt acggatggca   2340
   tgacagtaag agaattatgc agtgctgcca taaccatgag tgataacact gcggccaact   2400
   tacttctgac aacgatcgga ggaccgaagg agctaaccgc ttttttgcac aacatggggg   2460
   atcatgtaac tcgccttgat cgttgggaac cggagctgaa tgaagccata ccaaacgacg 2520
   agcgtgacac cacgatgcct gtagcaatgg caacaacgtt gcgcaaacta ttaactggcg 2580
   aactacttac tctagcttcc cggcaacaat taatagactg gatggaggcg gataaagttg 2640
   caggaccact tctgcgctcg gcccttccgg ctggctggtt tattgctgat aaatctggag 2700
   ccggtgagcg tgggtctcgc ggtatcattg cagcactggg gccagatggt aagccctccc 2760
   gtatcgtagt tatctacacg acggggagtc aggcaactat ggatgaacga aatagacaga 2820
   tcgctgagat aggtgcctca ctgattaagc attggtaact gtcagaccaa gtttactcat 2880
   atatacttta gattgattta aaacttcatt tttaatttaa aaggatctag gtgaagatcc 2940
   tttttgataa tctcatgacc aaaatccctt aacgtgagtt ttcgttccac tgagcgtcag 3000
   accccgtaga aaagatcaaa ggatcttctt gagatccttt ttttctgcgc gtaatctgct 3060
   gcttgcaaac aaaaaaacca ccgctaccag cggtggtttg tttgccggat caagagctac 3120
   caactctttt tccgaaggta actggcttca gcagagcgca gataccaaat actgttcttc 3180
   tagtgtagcc gtagttaggc caccacttca agaactctgt agcaccgcct acatacctcg 3240
   ctctgctaat cctgttacca gtggctgctg ccagtggcga taagtcgtgt cttaccgggt 3300
   tggactcaag acgatagtta ccggataagg cgcagcggtc gggctgaacg gggggttcgt 3360
   gcacacagcc cagcttggag cgaacgacct acaccgaact gagataccta cagcgtgagc 3420
   tatgagaaag cgccacgctt cccgaaggga gaaaggcgga caggtatccg gtaagcggca 3480
   gggtcggaac aggagagcgc acgagggagc ttccaggggg aaacgcctgg tatctttata 3540
   gtcctgtcgg gtttcgccac ctctgacttg agcgtcgatt tttgtgatgc tcgtcagggg 3600
   ggcggagcct atggaaaaac gccagcaacg cggccttttt acggttcctg gccttttgct 3660
   ggccttttgc tcacatagtc atgccccgcg cccaccggaa ggagctgact gggttgaagg 3720
   ctctcaaggg catcggtcga gatcccggtg cctaatgagt gagctaactt acattaattg 3780
   cgttgcgctc actgcccgct ttccagtcgg gaaacctgtc gtgccagctg cattaatgaa 3840
   tcggccaacg cgcggggaga ggcggtttgc gtattgggcg ccagggtggt ttttcttttc 3900
   accagtgaga cgggcaacag ctgattgccc ttcaccgcct ggccctgaga gagttgcagc 3960
   aagcggtcca cgctggtttg ccccagcagg cgaaaatcct gtttgatggt ggttaacggc 4020
   gggatataac atgagctgtc ttcggtatcg tcgtatccca ctaccgagat atccgcacca 4080
   acgcgcagcc cggactcggt aatggcgcgc attgcgccca gcgccatctg atcgttggca 4140
   accagcatcg cagtgggaac gatgccctca ttcagcattt gcatggtttg ttgaaaaccg 4200
   gacatggcac tccagtcgcc ttcccgttcc gctatcggct gaatttgatt gcgagtgaga 4260
   tatttatgcc agccagccag acgcagacgc gccgagacag aacttaatgg gcccgctaac 4320
   agcgcgattt gctggtgacc caatgcgacc agatgctcca cgcccagtcg cgtaccgtct 4380
   tcatgggaga aaataatact gttgatgggt gtctggtcag agacatcaag aaataacgcc 4440
   ggaacattag tgcaggcagc ttccacagca atggcatcct ggtcatccag cggatagtta    4500
   atgatcagcc cactgacgcg ttgcgcgaga agattgtgca ccgccgcttt acaggcttcg    4560
   acgccgcttc gttctaccat cgacaccacc acgctggcac ccagttgatc ggcgcgagat    4620
   ttaatcgccg cgacaatttg cgacggcgcg tgcagggcca gactggaggt ggcaacgcca    4680
   atcagcaacg actgtttgcc cgccagttgt tgtgccacgc ggttgggaat gtaattcagc    4740
   tccgccatcg ccgcttccac tttttcccgc gttttcgcag aaacgtggct ggcctggttc    4800
   accacgcggg aaacggtctg ataagagaca ccggcatact ctgcgacatc gtataacgtt    4860
   actggtttca cattcaccac cctgaattga ctctcttccg ggcgctatca tgccataccg    4920
   cgaaaggttt tgcgccattc gatggtgtcc gggatctcga cgctctccct tatgcgactc    4980
   ctgcattagg aagcagccca gtagtaggtt gaggccgttg agcaccgccg ccgcaaggaa    5040
   tggtgtggcc gattaatcat aaatatgaaa aataattgtt gcatcacccg ccaatgcgtg    5100
   gcttaatgca catcaaattg tgagcggata acaatttgat gtgctagcgc atatccagtg    5160
   tagtaaggca agtcccttca agagttatcg ttgatacccc tcgtagtgca cattccttta    5220
   acgcttcaaa atctgtaaag cacgccatat cgccgaaagg cacacttaat tattaagagg    5280
   taatacacca tgaatcacaa agtgcat                                        5307
<210> 11
   <211> 3836
   <212> DNA
   <213> Artificial
<220>
   <223> pPCR/calB
<220>
   <221> misc_feature
   <222> (666)..(1619)
   <223> calB_syn codierende Sequenz
<400> 11
   ctaaattgta agcgttaata ttttgttaaa attcgcgtta aatttttgtt aaatcagctc     60
   attttttaac caataggccg aaatcggcaa aatcccttat aaatcaaaag aatagaccga     120
   gatagggttg agtgttgttc cagtttggaa caagagtcca ctattaaaga acgtggactc     180
   caacgtcaaa gggcgaaaaa ccgtctatca gggcgatggc ccactacgtg aaccatcacc     240
   ctaatcaagt tttttggggt cgaggtgccg taaagcacta aatcggaacc ctaaagggag     300
   cccccgattt agagcttgac ggggaaagcc ggcgaacgtg gcgagaaagg aagggaagaa     360
   agcgaaagga gcgggcgcta gggcgctggc aagtgtagcg gtcacgctgc gcgtaaccac     420
   cacacccgcc gcgcttaatg cgccgctaca gggcgcgtcc cattcgccat tcaggctgcg     480
   caactgttgg gaagggcgat cggtgcgggc ctcttcgcta ttacgccagc tggcgaaagg     540
   gggatgtgct gcaaggcgat taagttgggt aacgccaggg ttttcccagt cacgacgttg     600
   taaaacgacg gccagtgagc gcgcgtaata cgactcacta tagggcgaat tgggtaccga     660
   attcgctgcc gagcggtagc gatccggcgt ttagccagcc gaaaagcgtt ctggatgcgg    720
   gtctgacctg tcagggtgcg agcccgagca gcgttagcaa accgattctg ctggttccgg    780
   gcaccggcac caccggtccg cagagctttg atagcaactg gattccgctg agcacccagc    840
   tgggttatac cccgtgttgg attagcccgc cgccgtttat gctgaatgat acccaggtga    900
   acaccgaata tatggtgaac gcgatcaccg cgctgtatgc gggtagcggc aataataaac    960
   tgccggtgct gacctggagc cagggtggtc tggttgcgca gtggggtctg accttttttc   1020
   cgagcattcg cagcaaagtg gatcgtctga tggcgtttgc gccggattat aaaggcaccg   1080
   ttctggcggg tccgctggat gcgctggcgg ttagcgcgcc gagcgtttgg cagcagacca   1140
   ccggtagcgc gctgaccacc gcgctgcgta atgcgggtgg tctgacccag attgttccga   1200
   ccaccaatct gtatagcgcg accgatgaaa ttgttcagcc gcaggttagc aatagcccgc   1260
   tggatagcag ctacctgttc aacggcaaaa atgttcaggc gcaggcggtt tgtggtccgc   1320
   tgtttgtgat tgatcatgcg ggtagcctga ccagccagtt tagctatgtg gttggtcgta   1380
   gcgcgctgcg tagcaccacc ggccaggcgc gtagcgcgga ttatggtatc accgattgca   1440
   atccgctgcc ggcgaatgat ctgaccccgg aacagaaagt tgcggcggca gcgctgctgg   1500
   cgccggcagc ggcggccatt gttgcgggtc cgaaacagaa ttgtgaaccg gatctgatgc   1560
   cgtatgcgcg tccgtttgcg gttggtaaac gtacctgcag cggtattgtg accccgtaat   1620
   aacatatgcg agctccagct tttgttccct ttagtgaggg ttaattgcgc gcttggcgta   1680
   atcatggtca tagctgtttc ctgtgtgaaa ttgttatccg ctcacaattc cacacaacat   1740
   acgagccgga agcataaagt gtaaagcctg gggtgcctaa tgagtgagct aactcacatt   1800
   aattgcgttg cgctcactgc ccgctttcca gtcgggaaac ctgtcgtgcc agctgcatta   1860
   atgaatcggc caacgcgcgg ggagaggcgg tttgcgtatt gggcgctctt ccgcttcctc   1920
   gctcactgac tcgctgcgct cggtcgttcg gctgcggcga gcggtatcag ctcactcaaa   1980
   ggcggtaata cggttatcca cagaatcagg ggataacgca ggaaagaaca tgtgagcaaa   2040
   aggccagcaa aaggccagga accgtaaaaa ggccgcgttg ctggcgtttt tccataggct   2100
   ccgcccccct gacgagcatc acaaaaatcg acgctcaagt cagaggtggc gaaacccgac   2160
   aggactataa agataccagg cgtttccccc tggaagctcc ctcgtgcgct ctcctgttcc   2220
   gaccctgccg cttaccggat acctgtccgc ctttctccct tcgggaagcg tggcgctttc   2280
   tcatagctca cgctgtaggt atctcagttc ggtgtaggtc gttcgctcca agctgggctg   2340
   tgtgcacgaa ccccccgttc agcccgaccg ctgcgcctta tccggtaact atcgtcttga   2400
   gtccaacccg gtaagacacg acttatcgcc actggcagca gccactggta acaggattag   2460
   cagagcgagg tatgtaggcg gtgctacaga gttcttgaag tggtggccta actacggcta   2520
   cactagaagg acagtatttg gtatctgcgc tctgctgaag ccagttacct tcggaaaaag   2580
   agttggtagc tcttgatccg gcaaacaaac caccgctggt agcggtggtt tttttgtttg   2640
   caagcagcag attacgcgca gaaaaaaagg atctcaagaa gatcctttga tcttttctac 2700
   ggggtctgac gctcagtgga acgaaaactc acgttaaggg attttggtca tgagattatc 2760
   aaaaaggatc ttcacctaga tccttttaaa ttaaaaatga agttttaaat caatctaaag 2820
   tatatatgag taaacttggt ctgacagtta ccaatgctta atcagtgagg cacctatctc 2880
   agcgatctgt ctatttcgtt catccatagt tgcctgactc cccgtcgtgt agataactac 2940
   gatacgggag ggcttaccat ctggccccag tgctgcaatg ataccgcgag acccacgctc 3000
   accggctcca gatttatcag caataaacca gccagccgga agggccgagc gcagaagtgg 3060
   tcctgcaact ttatccgcct ccatccagtc tattaattgt tgccgggaag ctagagtaag 3120
   tagttcgcca gttaatagtt tgcgcaacgt tgttgccatt gctacaggca tcgtggtgtc 3180
   acgctcgtcg tttggtatgg cttcattcag ctccggttcc caacgatcaa ggcgagttac 3240
   atgatccccc atgttgtgca aaaaagcggt tagctccttc ggtcctccga tcgttgtcag 3300
   aagtaagttg gccgcagtgt tatcactcat ggttatggca gcactgcata attctcttac 3360
   tgtcatgcca tccgtaagat gcttttctgt gactggtgag tactcaacca agtcattctg 3420
   agaatagtgt atgcggcgac cgagttgctc ttgcccggcg tcaatacggg ataataccgc 3480
   gccacatagc agaactttaa aagtgctcat cattggaaaa cgttcttcgg ggcgaaaact 3540
   ctcaaggatc ttaccgctgt tgagatccag ttcgatgtaa cccactcgtg cacccaactg 3600
   atcttcagca tcttttactt tcaccagcgt ttctgggtga gcaaaaacag gaaggcaaaa 3660
   tgccgcaaaa aagggaataa gggcgacacg gaaatgttga atactcatac tcttcctttt 3720
   tcaatattat tgaagcattt atcagggtta ttgtctcatg agcggataca tatttgaatg 3780
   tatttagaaa aataaacaaa taggggttcc gcgcacattt ccccgaaaag tgccac 3836
<210> 12
   <211> 317
   <212> PRT
   <213> Candida antarctica
<220>
   <221> MISC_FEATURE
   <222> (1)..(342)
   <223> reifes Protein CAA83122 (ohne Signalsequenz)
<400> 12
<210> 13
   <211> 317
   <212> PRT
   <213> Candida antarctica
<220>
   <221> MISC_FEATURE
   <223> reifes Protein CAA83122 (ohne Signalsequenz) mit Austauschen T57A und A89T
<400> 13
   Leu Pro Ser Gly Ser Asp Pro Ala Phe Ser Gln Pro Lys Ser Val Leu
   1               5                   10                  15
   Asp Ala Gly Leu Thr Cys Gln Gly Ala Ser Pro Ser Ser Val Ser Lys
               20                  25                  30
   Pro Ile Leu Leu Val Pro Gly Thr Gly Thr Thr Gly Pro Gln Ser Phe
           35                  40                  45
   Asp Ser Asn Trp Ile Pro Leu Ser Ala Gln Leu Gly Tyr Thr Pro Cys
       50                  55                  60
   Trp Ile Ser Pro Pro Pro Phe Met Leu Asn Asp Thr Gln Val Asn Thr
   65                  70                  75                  80
   Glu Tyr Met Val Asn Ala Ile Thr Thr Leu Tyr Ala Gly Ser Gly Asn
                   85                  90                  95
   Asn Lys Leu Pro Val Leu Thr Trp Ser Gln Gly Gly Leu Val Ala Gln
               100                 105                 110
   Trp Gly Leu Thr Phe Phe Pro Ser Ile Arg Ser Lys Val Asp Arg Leu
           115                 120                 125
   Met Ala Phe Ala Pro Asp Tyr Lys Gly Thr Val Leu Ala Gly Pro Leu
       130                 135                 140
   Asp Ala Leu Ala Val Ser Ala Pro Ser Val Trp Gln Gln Thr Thr Gly
   145                 150                 155                 160
   Ser Ala Leu Thr Thr Ala Leu Arg Asn Ala Gly Gly Leu Thr Gln Ile
                   165                 170                 175
   Val Pro Thr Thr Asn Leu Tyr Ser Ala Thr Asp Glu Ile Val Gln Pro
               180                 185                 190
   Gln Val Ser Asn Ser Pro Leu Asp Ser Ser Tyr Leu Phe Asn Gly Lys
           195                 200                 205
   Asn Val Gln Ala Gln Ala Val Cys Gly Pro Leu Phe Val Ile Asp His
       210                 215                 220
   Ala Gly Ser Leu Thr Ser Gln Phe Ser Tyr Val Val Gly Arg Ser Ala
   225                 230                 235                 240
   Leu Arg Ser Thr Thr Gly Gln Ala Arg Ser Ala Asp Tyr Gly Ile Thr
                   245                 250                 255
   Asp Cys Asn Pro Leu Pro Ala Asn Asp Leu Thr Pro Glu Gln Lys Val
               260                 265                 270
   Ala Ala Ala Ala Leu Leu Ala Pro Ala Ala Ala Ala Ile Val Ala Gly
           275                 280                 285
   Pro Lys Gln Asn Cys Glu Pro Asp Leu Met Pro Tyr Ala Arg Pro Phe
       290                 295                 300
   Ala Val Gly Lys Arg Thr Cys Ser Gly Ile Val Thr Pro
   305                 310                 315

## Beschreibung

Die vorliegende Erfindung betrifft für Triacylglycerol-Lipasen codierende Nukleinsäuren, solche Nukleinsäuren umfassende Vektor, Wirtszellen, welche solche Nukleinsäuren oder Vektoren umfassen, Verfahren zur Expression von Triacylglycerol-Lipasen in Prokaryoten, Verfahren zum Nachweis und zur Herstellung von Triacylglycerol-Lipasen, dadurch erhältliche Triacylglycerol-Lipasen, sowie die Verwendung von Triacylglycerol-Lipase-codierenden Nukleinsäuren, Vektoren und rekombinanten Wirtszellen für die genannten Verfahren.

### Hintergrund der Erfindung

Triacylglycerol-Lipasen (EC 3.1.1.3) sind geschätzte und effiziente Katalysatoren für verschiedenste industrielle Anwendungen, wie z.B. in der Detergens-Industrie, Oleochemie, Nahrungsmittelindustrie und bei der Herstellung von Feinchemikalien (Schmid 1998). Lipasen sind Carbonsäureester-Hydrolasen, welche die Hydrolyse wie auch die Synthese von Triglyceriden und anderen meist hydrophoben Estern katalysieren. Alle Triacylglycerinlipasen, deren dreidimensionale Kristallstruktur aufgeklärt wurde, sind Angehörige der α/β-Hydrolasefaltungs-Proteinfamilie, welche eine ähnliche Gesamtarchitektur aufweisen (Ollis 1992).

Die Klonierung und Expression verschiedener Lipasen wurde beschrieben (Sharma, 2001), ebenso wie die Klonierung und Überexpression einer Lipase aus *Archeoglobus fulgidus* (Rusnak, 2005) und die Überexpression einer Lipase aus *Pseudomonas alcaligenes* im gleichen Organismus (Gerritse, 1998).

*Candida antarctica*-Lipase B (CalB) ist ein effizienter Katalysator für viele Reaktionen und wird beispielsweise für stereoselektive Transformationen und die Polyester-synthese verwendet (Anderson 1998). CalB hat ein Lösungsmittel-zugängliches aktives Zentrum (Uppenberg 1994) und zeigt keinelnterphasen-Aktivierung (Martinelle et al., 1995). Das aktive Zentrum ist ein enger Trichter und CalB weist aus diesem Grund eine höhere Aktivität gegenüber Carbonsäureestem, wie beispielsweise Ethyloctanoat, auf als gegenüber Triglyceriden (Martinelle 1995). Die Tatsache, dass die Aktivität von CalB in organischen Medien denen in Wasser vergleichbar ist, sowie insbesondere die hohe Enantioselektivität von CalB gegenüber sekundären Alkoholen machen dieses Enzym zu einer der wichtigsten Lipasen, die gegenwärtig in der Biotechnologie verwendet werden.

In der Vergangenheit wurde CalB für Anwendungen im großindustriellen Maßstab hauptsächlich in *Aspergillus oryzae* exprimiert (Hoegh 1995). Für Forschungsanwendungen wurde das Enzym erfolgreich in den Hefen *Pichia pastoris* (Rotticci-Mulder et al. 2001), *Saccharomyces cerevisiae* (Zhang et al. 2003) und Hansenula *polymorpha* (Choi, 2004) exprimiert. Die Expression von CalB in dem einfach handhabbaren prokaryotischen Expressionssystem *Escherichia coli* (*E.coli*) scheiterte (Rotticci-Mulder 2003). Eine erstmalige Expression in *E.coli* gelang zwar später, führte aber nur zu geringen Ausbeuten an funktionaler CalB (Rusnak 2004). Dies ist bedauerlich, da *E.coli* im Vergleich mit anderen Expressionssystemen viele signifikante Vorteile aufweist und das schnelle und billige Hochdurchsatz-Screening großer Gen-Bibliotheken ermöglicht.

Kürzlich wurde die Modifizierung von CalB durch zufällige Mutagenese beschrieben (Chodorge et al., 2005). In der Literatur wurde weiterhin über mehrere Versuche berichtet, CalB für spezielle Anwendungen durch rationales Enzymdesign zu verbessern. Obwohl einige davon zu guten Ergebnissen führten (Patkar et al. 1998; Rotticci 2000), sind die Möglichkeiten des rationalen Enzymdesigns immer noch durch das mangelnde Verständnis der katalytischen Eigenschaften des Enzyms beschränkt.

Das bisher nicht gelöste Hauptproblem besteht jedoch nach wie vor in der unzureichenden Funktionalität von CalB bei Expression in *E.coli,* welche eine Voraussetzung für die Verbesserung von Enzymen durch gerichtete Evolution ist. Als Grund dafür wird die komplexe Tertiärstruktur des Enzyms gesehen, welche die Bildung von drei Disulfidbrücken erfordert, um eine funktionale Konformation zu gewährleisten. Die Herstellung eines solchen Proteins in *E.coli* oder anderen Prokaryoten ist mit Schwierigkeiten verbunden, weil die zelluläre Umgebung, die Faltungsmaschinerie und die Checkpoints der Faltungs-Qualitätskontrolle von Prokaryoten sich von denen der Eukaryoten unterscheiden (Baneyx und Mujacic 2004). In Übereinstimmung damit stellten die Erfinder bei anfänglichen Expressionsexperimenten von CalB in *E.coli* die Bildung von Einschlusskörpern und fehlende Aktivität von CalB fest (Ergebnisse nicht gezeigt).

Zusätzliche Probleme können auf der Ebene der Translation auftreten. Die Menge der tRNA-Spezies kann in den verschiedenen Organismen stark variieren. Dieses Problem kann durch Codon-Optimierung überwunden werden, und tatsächlich wurden die Expressionsgrade einiger eukaryotischer Proteine, z.B. einer Domäne des menschlichen Typ 1 Neurofibrominproteins, signifikant gesteigert (Hale 1998). Allerdings korreliert die Menge an funktionalem Enzym nicht direkt mit dem Expressionsgrad und daher auch nur konditional mit der Codon-Nutzung.

Zusätzlich zur tatsächlichen Gensequenz spielt der Promotor eine zentrale Rolle bei der Expressionseffizienz. In der Biotechnologie enthalten häufig verwendete Vektorsysteme, z.B. das pET Vektorsystem (Novagen), den T7-Promoter, der sie für die strenge Regulation der starken Überexprimierung heterologer Proteine in *E.coli* geeignet macht. Hohe Expressionsgrade heterolog exprimierter Enzyme führen allerdings sehr oft zu inkorrekt gefalteten Proteinen.

Es wurde gezeigt, dass kälteempfindliche Promotoren eine effiziente Genexpression bestimmter Proteine bei verringerten Temperaturen erleichtern können. Insbesondere wurde der Promotor des Haupt-Kälteschockgens cspA in den vergangenen Jahren verwendet (Goldstein et al. 1990). Wie aus Vergleichsstudien hervorgeht, ist die Expression löslichen Proteins in *E.coli* allerdings nach wie vor problematisch und hängt vom Protein sowie vom Ursprungsorganismus des Proteins ab (Qing et al. 2004).

Die zelluläre Umgebung kann ebenfalls einen Einfluss auf die Ausbeute an funktionalem, d.h. enzymatisch aktivem Protein ausüben. Es wurde berichtet, dass Mutationen in den Genen der Glutathion-Reduktase (*gor*) und Thioredoxin-Reduktase (*trxB*) zu verstärkter Ausbildung von Disulfidbrücken in Proteinen bei Expression im Cytoplasma von *E.coli* führen können (Prinz et al. 1997).

Ein Ansatz zur Verbesserung der Ausbeute an löslichen Proteinen im Cytoplasma von *E. coli* umfasst die Co-Expression molekularer Chaperone, die an der *de novo-*Proteinfaltung beteiligt sind. So wurde in der Vergangenheit berichtet, dass die Überexpression der Chaperone DnaK-DnaJ oder Trigger Faktor (TF) die Löslichkeit ausgewählter Proteine bei Expression in *E.coli* erhöhte (Nishihara et al. 2000). Für Zielproteine > 60 kD wurden gute Ergebnisse berichtet. Ein weiterer Mechanismus auf der Basis von GroEL-GroES kann nützlich sein für Zielproteine, die kleiner sind als etwa 60 kD (Baneyx und Mujacic 2004).

Ungeachtet der in der Vergangenheit erzielten Fortschritte hinsichtlich der funktionalen Expression von heterologen Proteinen in *E.coli* ist eine erfolgreiche Expression jedoch nach wie vor nicht vorhersagbar, sondern hängt stark vom jeweils verwendeten Protein ab.

Bisher liegen keine Berichte über die Erhöhung der funktionalen Expression von rekombinanter Triacylglycerol-Lipase, wie insbesonder CalB, vor.

Der vorliegenden Erfindung lag somit die Aufgabe zugrunde, für Triacylglycerol-Lipasen codierende Nukleinsäuren sowie Verfahren zu deren verbesserter funktionaler Expression in Prokaryoten bereitzustellen. Eine weitere Aufgabe bestand in der Bereitstellung eines Nachweisverfahrens für Triacylglycerol-Lipasen, eines Verfahrens für das Screening solcher Triacylglycerol-Lipasen und von Verfahren zur Herstellung solcher Triacylglycerol-Lipasen.

### Kurze Beschreibung der Erfindung

Die der Erfindung zugrundeliegende Aufgabe wurde gelöst durch ein Verfahren zur Expression von Triacylglycerol-Lipasen, bei dem die erhöhte funktionale Expression der Proteine erreicht wird durch Expression in prokaryotischen, insbesondere *E.coli-*Wirtsstämmen, unter den im Folgenden näher beschriebenen speziellen Bedingungen.

Die Aufgabe wurde weiterhin gelöst durch Bereitstellung von codierenden Nukleotidsequenzen, welche hinsichtlich der Expression von Lipase B in Prokaryonten, insbesondere *E.coli,* optimiert sind.

Die Aufgabe wurde weiterhin gelöst durch ein Nachweisverfahren von Triacylglycerol-Lipasen, insbesondere CalB, der Verwendung des Nachweisverfahren zum Screenen von Triacylglycerol-Lipasen, und ein Verfahren zu deren Herstellung.

### Figurenbeschreibung

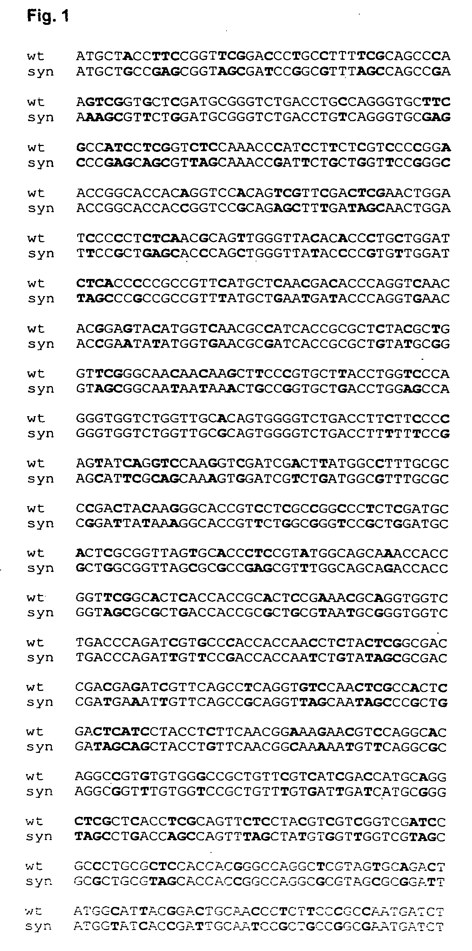

Fig. 1: Sequenzvergleich zwischen aus *C.antarctica* amplifizierten calB_wt und dem aus dem Vektor pPCR/calB stammenden synthetischen sequenzoptimierten Gen calB_syn. Unterschiede sind hervorgehoben.

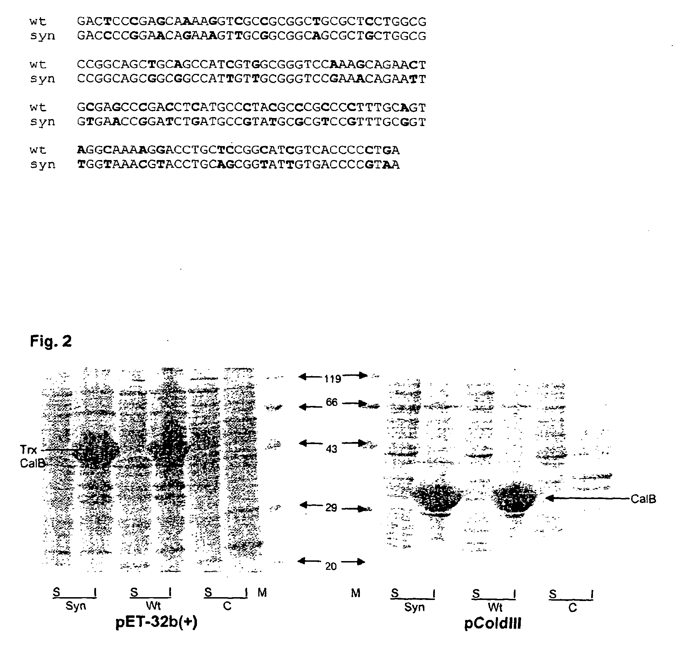

Fig. 2: SDS-PAGE-Auftrennung löslicher (S) und unlöslicher (I) Fraktionen, die bei 15°C unter Verwendung der Expressionsvektoren pET32-b(+) (in OrigaMi™ 2 (DE3)-Zellen) bzw. pColdIII (in Origami™ B-Zellen) in den CalB-Expressionsexperimenten erhalten wurden. CalB-Banden (33 kDa) und Trx-CalB-Fusionsprotein-Banden (45 kDa) sind mit Pfeilen markiert. M: Molekulargewichtsstandard. C: Fraktionen einer Kontrolle mit leerem Vektor.

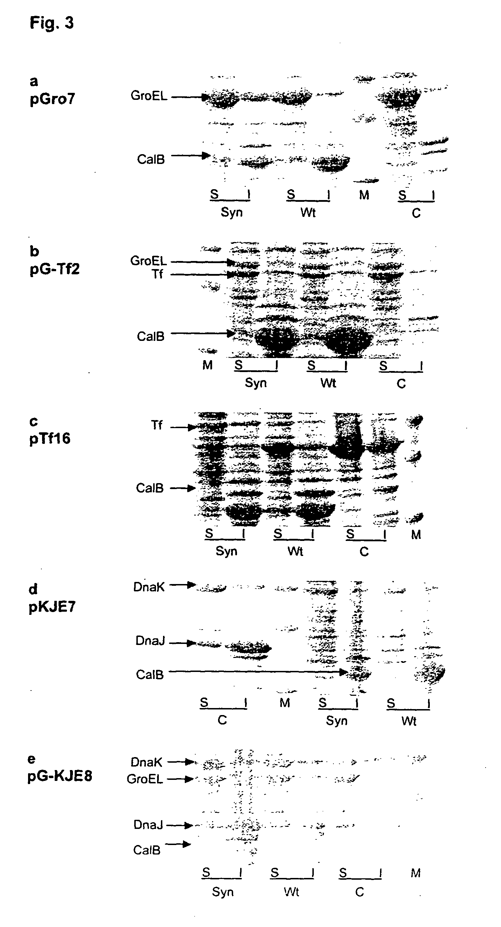

Fig. 3: SDS-PAGE-Auftrennung löslicher (S) und unlöslicher (I) Fraktionen, die durch Co-Expressionsexperimente von CalB unter Verwendung von pColdIII-Konstrukten mit unterschiedlichen Chaperon-Plasmiden (a: pGro7, b: pG-Tf2, c: pTf16, d: pKJE7, e: pG-KJE8) in Origami™ B-Zellen erhalten wurden. CalB (33 kDa) und Chaperone (GroEL: 60 kDa, Tf: 56 kDa, DnaK: 70 kDa, DnaJ: 40 kDa) sind mit Pfeilen markiert. M: Molekulargewichtsstandard (29, 43 und 66 kDa). C: Fraktionen von einer Kontrolle mit leerem Vektor.

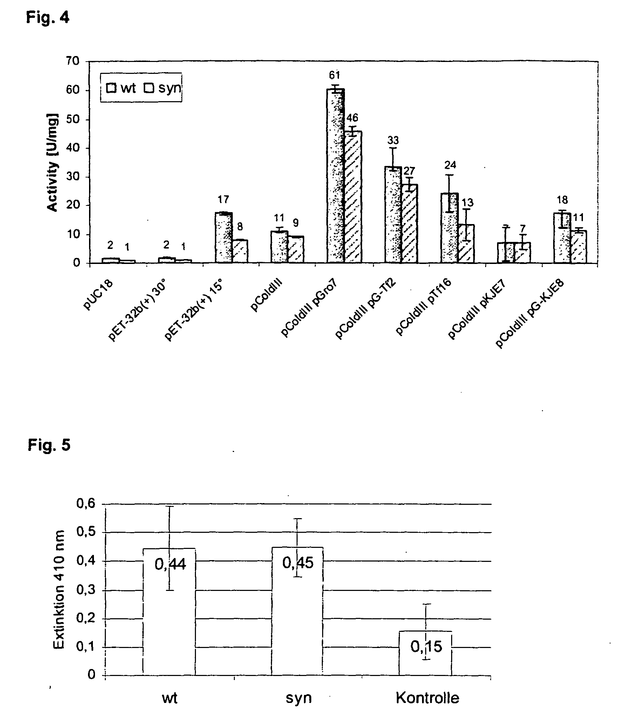

Fig. 4 : Hydrolytische Aktivität gegenüber Tributyrin von geklärten Zelllysaten aus *E.coli* Origami™ 2(DE3)-Zellen (im Fall von pET32b(+)-Expression) und Origami™ B-Zellen (alle anderen Konstrukte), welche die angegebenen Konstrukte tragen. Der Mittelwert und die Standardabweichung aus 4-6 unabhängigen Expressionsexperimenten sind angegeben.
Fig. 5 : Hydrolyse von pNPP in 96-Well-Miktotiterplatten durch geklärte Zelllysate von Origami B-Zellen, welche pCofdIII /calB_wt oder_syn) und GroES / GroEL(pGro7) enthalten. Im Fall CalB-exprimierender Zellen wurden 17 (calB_wt) and 18 (calB_syn) Wells untersucht. 6 Wells mit Origami™ B-Zellen, welche den leeren pColdIII-Vektor und pGro7 enthielten, wurden als Kontrollen verwendet. Die Werte waren durch Hintergrundextinktionswerte (Substrat ohne Zelllysat) normalisiert.

### Detaillierte Beschreibung der Erfindung

Ein erster Gegenstand der Erfindung betrifft ein Verfahren zur Expression funktionaler Triacylglycerol-Lipase in Prokaryoten, wobei man eine Triacylglycerol-Lipase codierende Nukleotidsequenz unter der Kontrolle eines induzierbaren Promotors in einem Thioredoxin-Reduktase-defizienten und Glutathion-Reduktase-defizienten *E.coli-*Stamm bei einer Temperatur exprimiert, die ausgewählt ist aus einem Bereich von 1°C bis 25°C. Die Nukleotidsequenz ist SEQ ID NO:1 (calB_syn) oder SEQ ID NO:2 (calB_wt) oder eine dazu homologe Nukleotidsequenz, bei der nicht mehr als 20% der Nukleotide verschieden sind von SEQ ID NO:1. Der induzierbare Promotor ist T7 (SEQ ID NO:3) oder ein durch Kälteschock induzierbarer Promotor.

Erfindungsgemäß werden unter Triacylglycerol-Lipasen Enzyme der Klasse E.C. 3.1.1.3 gemäß der IUBMB Enzym-Nomenklatur verstanden (http://www.iubmb.unibe.ch; http://www.chem.qmul.ac.uk/iubmb/enzyme/). Das erfindungsgemäße Verfahren eignet sich darüber hinaus insbesondere zur funktionalen Expression von Lipasen, die in ihrer funktionalen Form eine oder mehrere S-S-Brücken (Disulfidbrücken) benötigen, beispielsweise 1, 2, 3, 4, 5 oder 6 S-S-Brücken pro Peptidkette, wobei die S-S-Brücken zwischen schwefelhaltigen Aminosäuren der gleichen Peptidkette (intramolekular) und/oder schwefelhaltigen Aminosäuren verschiedener Peptidketten (intermolekular) ausgebildet sein können. Weitere Beispiele für Lipasen mit S-S-Brücken sind Lipase aus *Aspergillus oryzae* (Tsuchiya et al., 1996), Lipase aus *Penicillum camenbertii* (Yamaguchi et al., 1991), Lipase aus *Rhizomucor mihei* (Boel et al., 1988) und Lipase aus *Candida rugosa* (Longhi et al., 1992).

Die Expression erfolgt bei niedrigen Temperaturen. Im Rahmen dieser Erfindung werden unter niedrigen Temperaturen Raumtemperatur oder unter Raumtemperatur liegende Temperaturen verstanden, also Temperaturen von etwa 25°C bis 1°C, beispielsweise 25°C, 24°C, 23°C, 22°C, 21°C, 20°C, 19°C, 18°C, 17°C, 16°C, 15°C, 14°C, 13°C, 12°C, 11°C, 10°C, 9°C, 8°C, 7°C, 6°C, 5°C, 4°C, 3°C, 2°C, 1°C, oder zwischen diesen Werten liegende Temperaturen.

Gemäß weiteren Ausführungsformen ist die Temperatur für die Expression ausgewählt aus einem Bereich von 1°C bis 20°C, insbesondere einem Bereich von 10°C bis 20°C, beispielsweise 10°C, 11°C, 12°C, 13°C, 14°C, 15°C, 16°C, 17°C, 18°C, 19°C oder 20°C, und insbesondere einem Bereich von 13°C bis 16°C, beispielsweise 13°C, 14°C, 15°C oder 16°C. In einer speziellen Ausführungsform beträgt die für die Expression verwendete Temperatur etwa 15°C.

Ein Gegenstand der Erfindung betrifft die Expression in einem Thioredoxin-Reduktase-defizienten und/oder Gluthathion-Reduktase-defizienten *E.coli-*Stamm*.* Beispiele für solche Stämme sind Origami™ 2((DE3) und Origami™ B (Novagen, Darmstadt, Deutschland).

Ohne sich auf eine Theorie festzulegen, wird angenommen, dass diese Enzyme die Ausbildung von S-S-Brücken in Proteinen verhindern oder zur Reduktion bereits gebildeter S-S-Brücken beitragen. Das Fehlen eines oder mehrerer dieser Enzyme oder die Unterdrückung ihrer enzymatischen Aktivität stabilisiert somit die Konformation solcher S-S-Brücken enthaltender Proteine.

Gemäß einer speziellen Ausführungsform des erfindungsgemäßen Verfahrens ist die funktional exprimierte Triacylglycerol-Lipase Lipase B, das Genprodukt von calB aus *Candida antarctica.* Das calB-Gen wurde beschrieben (Uppenberget al., 1994) und seine Nukleotid- bzw. Proteinsequenz unter den Zugangsnummern Z30645 bzw. CAA83122.1 bei GenBank hinterlegt. Sofern nicht genauer bezeichnet, bedeutet hier calB eine Nukleotidsequenz dieser Zugangsnummer. Ein Beispiel für eine weitere Tri-acylglycerol-Lipase ist die Lipase B aus *Pseudozyma tsukubaensis* (Suen et al. 2004).

Gemäß einer weiteren speziellen Ausführungsform des erfindungsgemäßen Verfahrens ist die Triacylglycerol-Lipase codierende Sequenz calB_wt (SEQ ID NO:2). calB_wt entstammt einem früheren Projekt der Erfinder, bei dem CalB funktional in *Pichia pastoris* exprimiert wurde, und ist im pPICZαA/calB-Konstrukt enthalten (Rusnak 2004). In jenem Projekt zeigte das aus genomischer DNA von *Candida antarctica* amplifizierte calB-Gen zwei Veränderungen gegenüber der veröffentlichten CalB-Sequenz (CAA83122.1) auf Aminosäure-Ebene (T57A, A89T; SEQ ID NO:13). Die zwei Abweichungen zeigten sich in zwei unabhängigen Amplifizierungsansätzen, in denen das Gen aus zwei verschiedenen Extrakten genomischer DNA amplifiziert wurde. Aus diesem Grund sind sie höchstwahrscheinlich natürliche Variationen des Lipase-Gens. Die Lipase zeigte bei Expression in *Pichia pastoris* eine mit den veröffentlichten Werten des Wildtyp-CalB vergleichbare Aktivität, so dass die Erfinder in den früheren Projekten mit dem amplifizierten Gen weiterarbeiteten (Rotticci-Mulder et al., 2001; Rusnak 2004).

Gemäß einer weiteren speziellen Ausführungsform des erfindungsgemäßen Verfahrens ist die Triacylglycerol-Lipase codierende Sequenz calB_syn (SEQ ID NO:1). calB_syn stellt das Ergebnis einer Sequenzoptimierung dar. Sequenzoptimierungsstrategien sind dem Fachmann bekannt und können eine oder eine Kombination mehrerer Maßnahmen umfassen. Beispielsweise werden Codons für Aminosäuren so ausgewählt, dass sie den relativ am häufigsten im ausgewählten Wirt vorkommenden Transfer-RNAs entsprechen. Darüber hinaus kann es vorteilhaft sein, Regionen mit sehr hohem (> 80 %) oder niedrigem (< 30 %) GC-Gehalt oder bestimmte Sequenzmotive zu vermeiden, die einen Einfluss auf die Expression, also die Transkription der DNA und/oder die Translation der mRNA haben. Für die Herstellung von calB_syn wurde unter Verwendung der GeneOptimizer™-Technologie (GeneArt, Regensburg, Deutschland) die Codon-Verwendung optimiert und zusätzlich Regionen mit sehr hohem (> 80 %) oder niedrigem (< 30 %) GC-Gehalt falls möglich vermieden. Darüber hinaus wurden cis-wirkende Sequenzmotive wie beispielsweise interne TATA-Boxen, Chi-Stellen, ribosomale Andockungsstellen, ARE-, INS- und CRS-Sequenzelemente ebenso wie repetitive Sequenzen und RNA-Sekundärstrukturen vermieden. Das Gen unterscheidet sich in 253 Nukleotiden (26,5 %) von der calB_wt-Sequenz (Figur 1). Auf Aminosäure-Ebene kodiert das synthetische Gen das veröffentlichte Protein (CAA83122.1; SEQ ID NO:12).

Gemäß weiteren speziellen Ausführungsformen des erfindungsgemäßen Verfahrens sind die Triacylglycerol-Lipase codierenden Sequenzen Homologe von calB_wt oder calB_syn. calB von *C.antarctica,* insbesondere calB_wt und/oder calB_syn sowie deren Homologe, insbesondere funktionale Äquivalente codierende Homologe, stellen in jedem hier beschriebenen Zusammenhang bevorzugte Vertreter für Triacylglycerol-Lipasen codierende Nucleotidsequenzen dar.

Unter einer homologen Nukleotidsequenz oder einer homologen Nukleinsäure oder einem Homologen wird erfindungsgemäß verstanden, dass nicht mehr als 20%, 15%, 10%, 5%, 4%, 3%, 2% oder 1% der Nukleotide beim Vergleich mit einer Referenznukleotidsequenz oder Referenznukleinsäure unterschiedlich sind. Beispielsweise ist eine zu SEQ ID NO:1 homologe Sequenz hinsichtlich nicht mehr als 40% der Nukleotide, insbesondere nicht mehr als, 20%, 15% oder 10% der Nukleotide verschieden von SEQ ID NO:1.

Homologe Nuklotidsequenzen stellen insbesondere solche Sequenzen dar, die mit oben genannten Referenznukleotidsequenzen unter "stringenten Bedingungen" hybridisieren. Unter dieser Eigenschaft versteht man die Fähigkeit eines Poly- oder Oligonukleotids unter stringenten Bedingungen an eine nahezu komplementäre Sequenz zu binden, während unter diesen Bedingungen unspezifische Bindungen zwischen weniger komplementären Partnern unterbleiben. Dazu sollten die Sequenzen zu 70-100%, vorzugsweise zu 75%, 80%, 85% oder 90% bis 100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1 x SSC-Puffer mit 0,1 % SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z.B. in Ausubel et al. (1989) beschrieben (Kapitel 6.3.1-6.3.6). Homologe Nukleinsäuren lassen sich beispielsweise bei der Durchmusterung von genomischen oder cDNA-Banken auffinden und gegebenenfalls daraus mit geeigneten Primern mittels PCR vermehren und anschließend beispielsweise mit geeigneten Sonden isolieren.

Homologe der erfindungsgemäßen Triacylglycerol-Lipasen, insbesondere der erfindungsgemäßen Lipasen B aus *Candida antarctica,* können durch Screening kombinatorischer Banken von Mutanten, wie z.B. Verkürzungsmutanten, identifiziert werden. Beispielsweise kann eine Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang 1983; Itakura et al.,1984; Ike et al., 1983).

Gemäß weiterer Ausführungsformen steht die Triacylglycerol-Lipase codierende und insbesondere erfindungsgemäßes calB codierende Nukleotidsequenz unter der Kontrolle des T7-Promotors, gemäß SEQ ID NO:3. Geeignete Vektoren, die eine Expression unter Kontrolle des T7-Promotors erlauben, sind dem Fachmann bekannt, beipielsweise das pET-Vektorsystem (Novagen), z.B. die Vektoren pET-32a-c(+). Erfindungsgemäß werden bereitgestellt calB_syn bzw. calB_wt in pET-32b(+) (SEQ ID NO:7 bzw. SEQ ID NO:8). Die Expression aus diesen Vektoren erfolgt bei Temperaturen gemäß obenstehender Definition. Erfindungsgemäß wurde überraschend gefunden, dass bei Expression von calB_wt oder calB_syn aus pET-Vektoren ohne Verwendung besonderer, durch Kälte induzierbarer Promotoren durch Inkubation bei niedrigen Temperaturen ein erhöhter Anteil an funktionalem Protein gebildet wurde.

Gemäß weiteren Ausführungformen steht die Triacylglycerol-Lipase codierende und insbesondere erfindungsgemäßes calB codierende Nukleotidsequenz unter der Kontrolle eines durch Kälteschock induzierbaren Promotors. Im Rahmen der vorliegenden Erfindung wird unter Kälteschock verstanden, dass der Promotor niedrigen Temperaturen ausgesetzt wird. Ein geeigneter durch Kälteschock induzierbarer Promotor ist der Promotor des Haupt-Kälteschockgens cspA von *E.coli* (SEQ ID NO:4) (Goldstein et al., 1990). Diesen Promotor enthaltende Expressionsvektoren, die eine Klonierung eines gewünschten Zielgens über übliche Verfahren ermöglichen, sind dem Fachmann bekannt, beispielsweise die Vektoren pCOLD in ihren verschiedenen Variationen von Takara Bio Inc., Japan (Takara 2003). Erfindungsgemäß werden bereitgestellt calb_syn bzw. calB_wt in pCOLDIII (SEQ ID NO:9 bzw. SEQ ID NO:10). Es wurde überraschend gefunden, dass bei Expression aus diesen Vektoren in Origami™ 2(DE3)-Zellen und Origami™ B-Zellen eine erhöhte Menge an funktionalem Protein gebildet wird. Die Induktion des Kälteschock-Promotors erfolgt durch Inkubation bei niedrigen Temperaturen und gegebenenfalls unter Zugabe weiterer für die Expression erforderlicher Faktoren (beispielsweise IPTG bei Genen, die unter der Kontrolle des lac-Operators stehen). Eine kontrollierte Einstellung niedriger Temperaturen kann gegebenenfalls durch vorherige Inkubation (beispielsweise 30-minütige Inkubation) auf Eis erleichtert werden. Dem Fachmann sind weitere, durch Kälte induzierbare Promotoren bekannt, die beispielsweise beschrieben sind in Qoronfleh et al., 1992, Nakashima et al. 1996 oder Giladi et al.1995.

Ohne auf eine Theorie festgelegt zu sein, wird angenommen, dass eine Strategie zur erhöhten funktionalen Expression von Triacylglycerol-Lipasen, insbesondere CalB und deren funktionalen Äquivalenten, darin besteht, die Expression des Proteins im wesentlichen nur bei niedrigen Temperaturen zuzulassen. Erfolgt eine Expression bei darüberliegenden Temperaturen, so kann sich falsch gefaltetes Protein bilden, das als Kristallisationskeim fungiert, der die Bildung von funktionalem Protein stört, selbst wenn die Expression später unter Bedinungen erfolgt, die normalerweise zu funktionalem Protein führen (beispielsweise niedrige Temperaturen). Eine erfindungsgemäße Umsetzung dieser Strategie umfasst die Expression unter Kontrolle von Promotoren, die eine nennenswerte Expression nur bei niedrigen Temperaturen zulassen (beispielsweise den in pCOLD-Vektoren enthaltenen Promotoren), wobei die Expression gegebenenfalls zusätzlich kontrolliert wird durch Repressoren der Transkription (beispielsweise das Genprodukt von lacI, welches bei Fehlen von IPTG die Transkription verhindert und diese bei Gegenwart von IPTG zulässt). Eine weitere Umsetzung besteht darin, Promotoren, insbesondere starke Promotoren, zu verwenden, deren Transkriptionsaktivität streng kontrolliert werden kann, und eine Transkription von diesen Promotoren nur bei niedrigen Temperaturen zuzulassen. Neben pET-Vektoren, die T7-Promotoren umfassen, sind dem Fachmann wietere Promotoren bzw. Kombinationen von Promotoren und regulativen Elementen (beispielsweise Repressoren) bekannt, z.B. C1-regulierte Promotoren (Schofield et al., 2002), der PltetO-1-Promotor (Lutz und Bujard, 1997) oder *rha*T-Promotoren (Giacalone et al. 2006).

Die Inkubationsdauer wird für jedes verwendete Vektorsystem so gewählt, dass eine maximale Menge an funktionalem Protein gebildet wird, und kann von Fachmann über Routineversuche für das jeweils aus einer gegebenen Nukleinsäure zu exprimierende Protein leicht ermittelt werden. Übliche Zeiträume sind 1 bis 48 Stunden, beispielsweise 8, 12, 16 und 24 Stunden.

Gemäß einer weiteren Ausführungsform der erfindungsgemäßen Verfahrens werden gleichzeitig mit der Triacylglycerol-Lipase codierenden und insbesondere der calB codierenden Nukleotidsequenz ein oder mehrere Chaperone exprimiert. Die Chaperone sind beispielsweise ausgewählt unter GroES, GroEL, DnaK, DnaJ, GrpE und Trigger Factor (TF) von *E.coli.* Eine Expression ist in beliebigen Kombinationen möglich, insbesondere werden die jedoch co-exprimiert die Kombinationen GroEL und GroES; oder DnaK, DnaJ und GrpE; oder DnaK, DnaJ, GrpE, GroES und GroEL; oder es wird Trigger Factor, optional zusammen mit GroES und GroEL co-exprimiert. Die Chaperone können gemeinsam mit der für die Triacylglyderol-Lipase codierenden Nukleotidsequenz von einem Vektor exprimiert werden. Alternativ können die Triacylglycerol-Lipase codierende Nukleotidsequenz und die Chaperon(e) codierende(n) Nukleotidsequenz(en) von getrennten Vektoren exprimiert werden. Geeignete Vektoren sind im kommerziell erhältlichen "Chaperone Plasmid Set" enthalten, das die Plasmide pG-KJE8, pGro7, pKJE7, pG-Tf2 und pTf16, umfasst (Takara Biomedicals, Japan, Takara 2003b).

Ein weiterer Gegenstand der Erfindung betrifft ein Verfahren zum Nachweis von Triacylglycerol-Lipasen, wobei man
i) ein Protein, für das man eine Triacylglycerol-Lipase Aktivität vermutet, gemäß einem der oben genannten erfindungsgemäßen Verfahren exprimiert,
ii) das Expressionsprodukt mit einem von Triacylglycerol-Lipase hydrolysierbaren Substrat in Kontakt bringt, und
iii) die Hydrolyseaktivität bestimmt.

Dieses Verfahren ist geeignet für das Auffinden, d.h. Screenen neuer Triacylglycerol-Lipasen, insbesondere solcher mit einer der Lipase B von *Candida antarctica* vergleichbaren Enzymaktivität. Für den Fachmann ist es weiterhin ersichtlich, dass das Verfahren analog angewendet werden kann für Lipasen, in ihrer funktionalen Form eine oder mehrere S-S-Brücken benötigen, beispielsweise 1, 2, 3, 4, 5 oder 6 S-S-Brücken pro Peptidkette, wobei die S-S-Brücken zwischen schwefelhaltigen Aminosäuren der gleichen Peptidkette (intramolekular) und/oder schwefelhaltigen Aminosäuren verschiedener Peptidketten (intermolekular) ausgebildet sein können.

Geeignete hydrolysierbare Substrate sind dem Fachmann bekannt, der Nachweis der Hydrolyseaktivität kann ebenfalls in fachüblicher Weise erfolgen. Beispielsweise führt Tributyrin bei Zugabe zu Agarplatten in geeigneten Konzentrationen (z.B. 1 %) zu deren Trübung, die bei enzymatischer Hydrolyse verschwindet. Weitere geeignete Substrate sind Verbindungen, deren hydrolytische Spaltung zu einer beispielsweise photometrisch nachweisbaren Farbveränderung führt (z.B. p-Nitrophenylpalmitat). Die Hydrolyseaktivität kann weiterhin durch enzymatische Spaltung von Carbonsäureestern im pH-stat Assay bestimmt werden. Dabei wird das durch die freiwerdenden Carbonsäuren bedingte Absinken des pH-Werts durch Titration mit NaOH konstant gehalten. Der Verbrauch an NaOH, der proportional ist zur Menge an freigesetzer Carbonsäure, gibt somit einen Aufschluss über die Hydrolyseaktivität des Enzyms. Hinsichtlich der oben genannten Nachweisverfahren wird auf Rusnak, 2004 (Kapitel 8.4.2) verwiesen, auf das vollumfänglich Bezug genommen wird.

Weitere, durch Triacylglycerol-Lipase hydrolysierbare Substrate können ermittelt werden, indem man versucht, ein in Frage kommendes Substrat über dem Fachmann bekannte Triacylglycerol-Lipasen (beispielsweise CalB mit der Sequenz CAA83122.1) zu hydrolysieren. Lässt sich eine Hydrolyse feststellen, so kann das Substrat in dem erfindungsgemäßen Verfahren zum Nachweis von Triacylglycerol-Lipasen verwendet werden.

Wenngleich das oben beschriebene Nachweisverfahren in fachüblichen Petrischalen oder Gefäßen für die Zellkultur durchgeführt werden kann, so ist in einer speziellen Ausführungsform die Verwendung von Mikrotiterplatten vorgesehen. Beispielsweise kann die Expression des Proteins mit vermuteter Triacylglycerol-Lipase-Aktivität bereits in der Mikrotiterplatte erfolgen, etwa durch Kultivierung der Prokaryoten in der Mikrotiterplatte und Induzierung der Expression des Proteins. Weiterhin kann auch der Nachweis der Hydrolyseaktivität in der Mikrotiterplatte erfolgen, wobei je nach den Parametern der vorliegenden Kultur der Nachweis ohne vorherige Abtrennung der Mikroorganismen erfolgend kann. Beispielsweise kann bei geringen Zelldichten in den jeweiligen Wells der Mikrotiterplatte eine direkte Bestimmung der Hydrolyseaktivität durch Änderung der Trübungseigenschaften des Mediums oder Umsetzung eines photometrischen Substrats bestimmt werden, ohne dass die jeweiligen Messwerte durch eine hohe Zelldichte verfälscht würden. Alternativ können die prokaryotischen Zellen nach Expression des in das Kulturmedium freigesetzten Proteins mit vermuteter Triacylglycerol-Lipase-Aktivität vom Medium abgetrennt werden (beispielsweise durch Sedimentation der Zellen über Zentrifugation und Entfernen des Überstandes oder sofortiges Entfernen des Mediums im Falle adhärent an der Well-Oberfläche wachsender oder trägergebundener Zellen) und das Protein mit vermuteter Triacylglycerol-Lipase enthaltende Medium zur Bestimmung der Hydrolyseaktivität in neue Mikrotiterplatten überführt werden.

Gemäß einer speziellen Ausführungsform erfolgt die Expression des Proteins mit vermuteter Triacylglycerol-Lipase-Aktivität in Gegenwart eines hydrolysierbaren Substrats, beispielsweise eines in einem Kulturmedium gelösten Substrats. Gemäß einer weiteren Ausführungsform können Prokaryoten, die ein Protein mit vermuteter Triacylglycerol-Lipase-Aktivität exprimieren, beispielsweise auf einem Nährboden kultiviert werden, der ein hydrolysierbares Substrat enthält. Besonders geeignet sind in diesem Zusammenhang Agarnährböden, die durch ihren Gehalt an Tributyrin trüb sind. Bei Expression einer hydrolytisch aktiven Triacylglycerol-Lipase, beispielsweise nach Induktion des Triacylglycerol-Lipase codierenden Gens durch chemische Substanzen oder Temperaturveränderung je nach verwendetem Expressionsvektor, erfolgt eine Spaltung des Tributyrins und dadurch eine Klärung des trüben Agars. An Stellen des Agars mit ausreichender Triacylglycerol-Lipase-Aktivität entsteht somit ein Hof, der visuell oder automatisiert über geeignete Bildverarbeitungssysteme (z.B. Quantimet 500 Qwin; Leica, Cambridge, Großbritannien) erfasst werden kann und zur Identifizierung einer Triacylglycerol-Lipase-Aktivität hervorbringenden Bakterienkolonie dienen kann. Für den Fachmann erschließen sich weitere Modifikationen des Verfahrens, beispielsweise die Beimpfung von Tributyrin-Agar-gefüllten Wells von Mikrotiterplatten mit Bakteriensuspensionen in Verdünnungen, die pro Well einzelne Bakterienkolonien entstehen lassen, und anschließende visuelle oder automatisierte Bildauswertung.

Gemäß einer weiteren Ausführungsform wird das exprimierte Protein mit vermuteter Triacylglycerol-Lipase-Aktivität von den exprimierenden prokaryotischen Zellen, insbesondere *E.coli,* abgetrennt und anschließend in zellfreiem Zustand mit dem hydrolysierbaren Substrat in Kontakt gebracht. Dem Fachmann sind geeignete Verfahren zur Abtrennung bekannt, beispielsweise Sedimentation der Zellen durch Zentrifugation oder Abtrennung durch Filtration. Diese Ausführungsform weist den Vorteil auf, dass der nachfolgende Nachweis der Hydrolyseaktivität nicht durch die Anwesenheit der prokaryotischen Zellen beeinflusst wird.

Gemäß einer speziellen Ausführungsform erfolgt dieser Nachweis photometrisch durch Bestimmung der Abnahme eines hydrolysierbaren Substrats oder Zunahme des Hydrolyseprodukts. Dem Fachmann sind geeignete Substrate bekannt, beispielsweise p-Nitrophenylester wie p-Nitrophenylpalmitat oder p-Nitrophenylacetat. Die Parameter der photometrischen Messung können vom Fachmann ohne weiteres auf die verwendeten Substrate und Lösungen angepasst werden (beispielsweise empfiehlt sich bei Verwendung von p-Nitrophenylpalmitat (pNPP) die Messung der Zunahme der Extinktion bei 410 nm).

In einer speziellen Ausführungsform wird das erfindungsgemäße Nachweisverfahren zum Screenen (Screeing) mutagenisierter Proteine oder durch mutagenisierte Nukleinsäuren codierter Proteine auf Triacylglycerol-Lipase-Aktivität verwendet. Dabei kann es sich um Proteine handeln, denen durch Mutagenese Triacylglycerol-Lipase-Aktivität verliehen werden soll (beispielsweise durch Einbau von Sequenzmotiven, die als für diese Enzymaktivität wichtig erkannt wurden, in Proteine, die keine oder nur geringe Triacylglycerol-Lipase-Aktivität aufweisen), oder um Proteine mit bereits bekannter Triacylglycerol-Lipase-Aktivität, die durch Einführung einer oder mehrerer Mutationen modifiziert werden soll. Unter einer Mutagenese (die zu einer Mutation oder zu einer mutierten Nukleotidsequenz oder einem mutierten Protein führt) wird in Bezug auf Nukleotidsequenzen das Hinzufügen, Entfernen oder Austauschen wenigstens eines Nukleotids verstanden. In Bezug auf Proteine wird darunter das Hinzufügen, Entfernen oder Austauschen wenigstens einer Aminosäure verstanden. Bei einem mutagenisierten erfindungsgemäßen Protein handelt es sich insbesondere um ein solches Protein, dessen codierende Nukleotidsequenz die Lipase B aus *Candida antarctica* codierende Nukleotidsequenz (calB) umfasst, oder um ein Protein, dessen codierende Nukleotidsequenz wenigstens eine Mutation im Vergleich mit der codierenden Nukleötidsequenz einer Lipase B aus *Candida antarctica* (calB) aufweist, oder um ein Protein, dessen codierende Nukleotidsequenz homolog ist zur codierenden Nukleotidsequenz einer Lipase B aus *Candida antarctica* (calB), oder um ein Protein, das ein funktionales Äquivalent zu einer Lipase B aus *Candida antarctia* (CalB) darstellt. Bei all diesen Proteinen muss eine Mutagenisierung der das jeweilige Protein codierenden Nukleotidsequenz nicht zwangsläufig zu einer Veränderung der Aminosäuresequenz des exprimierten Proteins führen. Wie oben bereits erwähnt, sind dem Fachmann Verfahren bekannt, um die Nukleotidsequenz von Genen beispielsweise hinsichtlich der im für die Expression vorgesehenen Wirtsorganismus bevorzugten Codon-Nutzung (codon usage), der Vermeidung von mRNA-Sekundärstrukturen oder bestimmter Sequenzmotive zu optimieren (z.B. die GeneOptimizer™-Technologie von GeneArt, Regensburg, Deutschland). Diese Optimierungen können der Verbesserung der Expression sowohl auf Transkriptionsebene als auch auf Translationsebene dienen und gleichzeitig unter Ausnützung des degenerierten Codes, der für bestimmte Aminosäuren mehrere Basentripletts zulässt, zur Translation eines unveränderten Proteins dienen. In diesem Fall dient das erfindungsgemäße Nachweisverfahren der Kontrolle der Expression eines funktionalen Proteins. Andererseits können durch Mutagenese codierender Nucleotidsequenzen Proteine hergestellt werden, deren Aminosäuresequenz im Vergleich mit Proteinen, die von nicht-mutagenisierten Ausgangssequenzen exprimiert werden, unterschiedlich sind. In diesem Fall kann mit dem erfindungsgemäßen Nachweisverfahren überprüft werden, ob die durch die mutagenisierten Nukleotidsequenzen codierten Proteine eine im Vergleich mit den durch die nicht-mutageniserten Ausgangssequenzen codierten Proteine gleichbleibende oder eine modifizierte, beispielsweise eine verringerte (oder fehlende), eine erhöhte, oder eine hinsichtlich eines oder verschiedener Parameter der enzymatischen Aktivität veränderte Hydrolyseaktivität aufweist. Als solche Parameter, die insbesondere getestet werden können durch Wahl der Bedingungen, bei denen ein mutagenisertes Protein mit vermuteter Triacylglycerol-Lipase-Aktivität gemäß Schritt ii) des erfindungsgemäßen Nachweisverfahrens mit einem hydrolysierbaren Substrat in Kontakt gebracht wird, kommen beispielsweise in Frage die Substratspezifität, Enantioselektivitat oder Wechselzahl des Proteins sowie deren Abhängigkeit beispielsweise von Temperatur, pH, Ionenkonzentration und/oder Anwesenheit möglicher Inhibitoren oder Aktivatoren.

Die Mutagenese durch gezielte Veränderungen der Nukleotidsequenz, beispielsweise über die Polymerase-Kettenreaktion (PCR), sowie durch ungerichteter Veränderungen, beispielsweise über chemische Mutagenese, ist dem Fachmann bekannt und beispielsweise beschrieben in Roufa 1996 oder Kirchhoff und Desrosiers 1996.

Die oben beschriebene Verwendung des erfindungsgemäßen Nachweisverfahrens eignet sich insbesondere für das Screening von Genbanken. Beispielsweise kann eine Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang 1983; Itakura et al., 1984); Itakura et al. 1984; Ike et al.1983).

Die erfindungsgemäße Verwendung ist insbesondere geeignet zum Hochdurchsatz-Screening von Proben (High Throughput Screening), beispielsweise dem Screening einer Vielzahl von Proben nacheinander in kurzer Zeit oder dem gleichzeitigen Screening mehrerer paralleler Proben oder einer Kombination daraus. Sie stellt somit insbesondere ein geeignetes Verfahren für das Screening der oben beschriebenen Genbanken dar. Beispielsweise können diese auf Klone gescreent werden, die eine besonders hohe funktionale Expression von Triacylglycerol-Lipasen zeigen, oder solche, die Triacylglycerol-Lipasen mit veränderten Eigenschaften exprimieren. In diesem Zusammenhang ist insbesondere die Verwendung von Mikrotiterplatten zur Expression eines oder mehrerer untersuchter Protein und/oder zur Bestimmung von dessen/deren Hydrolyseaktivität vorteilhaft. Wie dem Fachmann bekannt ist, kann dabei ein hoher Probendurchsatz durch Automatisierung erreicht werden, beispielsweise durch Pipettierroboter, welche Protein mit vermuteter Hydrolaseaktivität enthaltende Überstände aus den zur Expression dieser Proteine verwendeten Wells der Mikrotiterplatten in für den Nachweis der Hydrolyseaktivität bestimmte Wells überführen oder Nachweisreagenzien in die solche Überstände enthaltenden Wells pipettieren. Weiterhin kann die photometrische Auswertung bei Verwendung von Mikrotiterplatten insbesondere durch Plattenlesegeräte erfolgen, welche automatisch die Extinktion der in den einzelnen Wells enthaltenen Lösungen messen. Bei Verwendung von Agarplatten kann der Nachweis von durch die Hydrolyseaktivität entstanden klaren Höfen oder anderweitig optisch nachweisbaren Höfen wie bereits oben beschrieben beispielsweise durch geeignete Bildverarbeitungssysteme (z.B. Quantimet 500 Qwin; Leica, Cambridge, Großbritannien) erfolgen.

Ein weiterer Gegenstand der Erfindung betrifft ein Verfahren zur Herstellung einer Triacylglycerol-Lipase (E.C. 3.1.1.3), wobei man über eines der oben beschriebenen Nachweisverfahren ein exprimiertes Protein mit Aktivität einer Triacylglycerol-Lipase (E.C. 3.1.1.3) nachweist, den dieses Protein exprimierenden Stamm unter Lipaseexprimierenden Bedingungen kultiviert und gegebenenfalls die exprimierte Lipase isoliert. Geeignete Isolierungsverfahren, die erforderlichenfalls durch Routineversuche für das jeweilige Protein angepasst werden können, sind dem Fachmann bekannt und beipielhaft unten beschrieben. Für den Fachmann ist es ersichtlich, dass das Verfahren analog angewendet werden kann für Lipasen, die in ihrer funktionalen Form eine oder mehrere S-S-Brücken benötigen, beispielsweise 1, 2, 3, 4, 5 oder 6 S-S-Brücken pro Peptidkette, wobei die S-S-Brücken zwischen schwefelhaltigen Aminosäuren der gleichen Peptidkette (intramolekular) und/oder schwefelhaltigen Aminosäuren verschiedener Peptidketten (intermolekular) ausgebildet sein können.

Unter "funktionalen Äquivalenten" versteht man erfindungsgemäß insbesondere Mutanten, welche sich in wenigstens einer Sequenzposition der Aminosäuresequenz einer zugrundegelegten Triacylgylcerol-Lipase, insbesondere einer beliebigen Lipase B, von dieser unterscheiden, aber trotzdem eine der oben genannten biologischen Aktivitäten besitzen, beispielsweise gleichbleibende, verringerte oder erhöhte Hydrolyseaktivität, oder veränderte Substratspezifität, Enantioselektivitat oder Wechselzahl sowie deren Abhängigkeit beispielsweise von Temperatur, pH, Ionenkonzentration, oder Anwesenheit möglicher Inhibitoren oder Aktivatoren.

"Funktionale Äquivalente" umfassen somit die durch eine oder mehrere Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man in diesem Zusammenhang sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Enzyme können an funktionalen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide, welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, insbesondere einzelne Domänen oder Sequenzmotive, der erfindungsgemäßen Polypeptide, welche z.B. die gewünschte biologische Aktivität aufweisen.

"Funktionale Äquivalente" sind außerdem Fusionsproteine, welche eine der natürlichen Racemasesequenzen oder davon abgeleitete funktionale Äquivalente und wenigstens eine weitere, davon funktional verschiedene, heterologe Sequenz in funktionaler N-oder C-terminaler Verknüpfung (d.h. ohne gegenseitigen wesentliche funktionale Beeinträchtigung der Fusionsproteinteile) aufweisen. Nichtlimitierende Beispiele für derartige heterologe Sequenzen sind z.B. Signalpeptide oder Enzyme.

Erfindungsgemäß umfasste "funktionale Äquivalente" sind zu den natürlichen Proteinen homologe Proteine. Diese besitzen wenigstens 60 %, vorzugsweise wenigstens 75%, insbesondere wenigstens 85 %, wie z.B. wenigstens 90%, 95% oder 99%, Homologie zu einer der natürlichen Aminosäuresequenzen, berechnet nach dem Algorithmus von Pearson und Lipman (Pearson und Lipman 1988). Eine prozentuale Homologie eines erfindungsgemäßen homologen Polypeptids bedeutet insbesondere prozentuale Identität der Aminosäurereste bezogen auf die Gesamtlänge einer der Aminosäuresequenzen eines erfindungsgemäßen Enzyms oder einer Enzymuntereinheit. Die vorliegende Erfindung umfasst insbesondere funktionale Äquivalente gemäß den vorstehend genannten Definitionen, die zusätzlich eine Homologie von wenigstens 60 %, vorzugsweise wenigstens 75%, insbesondere wenigstens 85 %, wie z.B. wenigstens 90%, 95% oder 99%, zur Ausgangssequenz aufweisen.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Funktionale Äquivalente können unter Anwendung der erfindungsgemäßen Verfahren ermittelt werden. Beispielsweise können Proteine, deren funktionale Äquivalenz zu Triacylglycerol-Lipasen und insbesondere CalB bestimmt werden soll, über das erfindungsgemäße Expressionsverfahren exprimiert und das erfindungsgemäße Nachweisverfahren untersucht werden. Exprimierte Proteine, die Hydrolyseaktivität aufweisen, insbesondere veränderte Hydrolyseaktivität im Vergleich mit der Triacylglycerol-Lipase bzw. CalB, stellen funktionale Äquivalente dar.

In diesem Zusammenhang können erfindungsgemäße Triacylglycerol-Lipase exprimierende Prokaryonten nach bekannten Verfahren kultiviert und fermentiert werden. Die Zellen werden dann, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie, Affinitätschromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper (1980) oder in Scopes (1981) beschrieben.

Ein weiterer Gegenstand der Erfindung betrifft eine für Triacylglycerol-Lipase codierende Nukleinsäure, insbesondere eine für Lipase B aus *Candida antarctica* codierende Nukleinsäure, die eine codierende Nukleotidsequenz gemäß SEQ ID NO:1 umfasst, oder eine Nukleotidsequenz, bei der nicht mehr als 20% der Nukleotide verschieden sind von SEQ ID NO:1. Ein weiterer Gegenstand der Erfindung betrifft einen rekombinanten Vektor, der eine solche für eine Triacylglycerol-Lipase codierende Nukleinsäure umfasst, welche mit wenigstens einer regulativen Nukleinsäuresequenz operativ verknüpft ist. Gemäß einer weiteren speziellen Ausführungsform umfasst die Triacylglycerol-Lipase codierende Nukleinsäuresequenz eine Nukleotidsequenz gemäß SEQ ID NO:1 oder SEQ ID NO:2. Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel 1990. Erfindungsgemäß wurde beispielsweise gefunden, dass insbesondere bei Co-Expression mit pGRO7 calB_syn (SEQ ID NO:1) und calB_wt (SEQ ID NO:2) erhöht funktional exprimiert wurden.

Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar. Geeignete Plasmide sind beispielsweise in *E. coli* pLG338, pACYC184, pBR322, , pUC19, pKC30, pRep4, pHS1, pKK223-3, pDHE19.2, pHS2, pPLc236, pMBL24, pLG200, pUR290, pIN-II¹¹³-B1, Igt11 oder pBdCl, in *Streptomyces* pIJ101, pIJ364, pIJ702 oder pIJ361, in *Bacillus* pUB110, pC194 oder pBD214, in *Corynebacterium* pSA77 oder pAJ667. Die genannten Plasmide stellen eine kleine Auswahl der möglichen Plasmide dar. Weitere Plasmide sind dem Fachmann wohl bekannt und können beispielsweise aus dem Buch Cloning Vectors (Hrsg. Pouwels P. H. et al. Elsevier, Amsterdam-New York-Oxford, 1985 , ISBN 0 444 904018) entnommen werden. Geeignete Vektoren sind solche, welche die funktionale Expression der für ein Protein mit Triacylgylcerol-Lipase-Aktivität codierenden Nukleotidsequenz ermöglichen, welche wiederum durch das oben beschriebene Nachweisverfahren bestimmt werden kann.

Spezielle Ausführungsformen umfassen den Vektor pET32b(+) sowie die weiteren Vertreter der pET-Vektorfamilie (Novagen 1999), insbesondere pET-32a-c, pET-41 a-c und pET-42a-c, und pCOLD III sowie weitere Vertreter der pCOLD-Vektorfamilie (zB. pCOLD I, pCOLD II, pCOLD IV, pCOLD TF, erhältlich beispielsweise von Takara Bio Europe S.A.S, Gennevilliers, Frankreich). Gemäß ganz besonderen Ausführungsformen werden als rekombinante Vektoren, die für eine Triacylglycerol-Lipase codierende Nukleinsäure umfassen, erfindungsgemäß bereitgestellt pET-32b(+)/calB_syn (SEQ ID NO:7, calB_syn in pET-32b(+)),
pET-32b(+)/calB_wt (SEQ ID NO:8, calB_wt in pET-32b(+)),
pCOLDIII/calB_syn (SEQ ID NO:9, calB_syn in pCOLDIII) und
pCOLDIII/calB_wt (SEQ ID NO:10, calB_wt in pCOLDIII).

Ein weiterer Gegenstand der Erfindung betrifft eine rekombinante Wirtszelle, die einen solchen Vektor oder eine für Triacylglycerol-Lipase codierende Nukleinsäure, welche eine codierende Nukleotidsequenz gemäß SEQ ID NO:1 oder eine dazu homologe Nukleotidsequenz umfasst. Die rekombinante Zelle kann insbesondere eine prokaryotische Zelle sein, insbesondere eine *E.coli*-Zelle. Weitere Beispiele für prokaryotische Zellen sind unter den gramnegativen Bakterien Vertreter der *Enterobacteriaceae* wie *Salmonella, Shigella, Serratia, Proteus, Klebsiella* oder *Enterobacter, Pseudomonas,* unter den grampositiven Bakterien beispielsweise Vertreter der Gattung *Bacillus,* z.B. *B.subtilis* und *B.licheniformis.*

Weitere Gegenstände der Erfindung betreffen die Verwendung erfindungsgemäßen codierenden Nukleinsäuresequenz, eines erfindungsgemäßen Vektors oder einer erfindungsgemäßen Wirtszelle zur Durchführung eines erfindungsgemäßen Verfahrens zur Expression einer funktionalen Triacylglycerol-Lipase, eines erfindungsgemäßen Verfahrens zum Nachweis einer funktionalen Triacylglycerol-Lipase, oder eines erfindungsgemäßen Verfahrens zur Herstellung einer funktionalen Triacylglycerol-Lipase. Es wurde gefunden, dass bei Verwendung der erfindungsgemäßen Vektoren in einem erfindungsgemäßen Expressionsverfahren, insbesondere bei niedrigen Inkubationstemperaturen, calB_wt und calB_syn überraschenderweise in pET-32b(+) (SEQ ID NO: 8 bzw. SEQ ID NO:7) oder in pCOLDIII (SEQ ID NO:10 bzw. SEQ ID NO:9) in erhöhtem Maße funktional exprimiert wurden. Erfindungsgemäß wurde weiterhin gefunden, dass bei Co-Expression mit pG-KJE8 und insbesondere Co-Expression mit pGRO7, pG-Tf2, oder pTf16 calB_syn (SEQ ID NO:1) und calB_wt (SEQ ID NO:2) in besonderem Maße erhöht funktional exprimiert wurden.

Für den Fachmann ist es ersichtlich, dass das erfindungsgemäße Verfahren zur Expression funktionaler Triacylglycerol-Lipasen, das Verfahren zu deren Nachweis und das Verfahren zu deren Herstellung auch auf weitere, von Triacylglycerol-Lipasen unterschiedliche Enzyme analog angewendet werden können. Beispielsweise können diese Verfahren auf Enzyme der EC-Klasse 3.1 (Ester-Hydrolasen) oder allgemein auf die der EC-Klasse 3 (Hydrolasen) adaptiert werden (http://www.iubmb.unibe.ch; http://www.chem.qmul.ac.uk/iubmb/enzyme/). Die erfindungsgemäßen Verfahren erstrecken sich dementsprechend auf alle Enzyme, die unter Verwendung der in diesen Verfahren offenbarten allgemeinen Prinzipien exprimiert, nachgewiesen oder hergestellt werden können, insbesondere solche Enzyme, für deren Funktionalität die Ausbildung einer oder mehrerer S-S-Brücken, beispielsweise 1, 2, 3, 4, 5 oder 6 S_S-Brücken, erforderlich ist, wobei die S-S-Brücken zwischen schwefelhaltigen Aminosäuren der gleichen Peptidkette (intramolekular) und/oder schwefelhaltigen Aminosäuren verschiedener Peptidketten (intermolekular) ausgebildet sein können.

### Beispiele

### I. Allgemeine Angaben

### Chemikalien

Soweit nicht anders angegeben, wurden alle Chemikalien von Fluka (Buchs, Schweiz), Sigma-Aldrich (Taufkirchen, Deutschland) und der Roth GmbH (Karlsruhe, Deutschland) bezogen.

### Stämme und Plasmide

*E. coli* DH5α wurde von Clontech (Heidelberg, Deutschland) bezogen. *E. coli* Origami™2(DE3), Origami™ B und das Plasmid pET-32b(+) wurden von Novagen bezogen (Darmstadt, Deutschland). Das Plasmid pUC18 wurde von MBI Fermentas (St. Leon-Rot, Deutschland), pcoldIII und das Chaperon-Plasmid Set, das die Plasmide pG-KJE8, pGro7, pKJe7, pG-Tf2 und pTf16 enthält, wurden von Takara (Otsu, Japan) bezogen. Das Konstrukt pPCR/calB, welches das Codon-optimierte calB-Gen enthielt, wurde von GENEART synthetisiert (Regensburg, Deutschland). Das Konstrukt pPICZαA/calB wurde früher beschrieben (Rusnak 2004).

### Klonierung von calB-Varianten

calB_wt wurde aus dem Templat-Konstrukt pPICZαA/calB unter Verwendung der Primer wt_pUC18_fw und wt_pUC18_rev (Tabelle 1) amplifiziert und anschließend in den Vektor pUC18 kloniert, wodurch das Konstrukt pUC18/calB_wt erhalten wurde. Für die Subklonierung in pET-32b(+) wurde das Lipasegen unter Vewendung der Primer wt_pET-32b(+)_fw und wt_pET32b(+)_rev (Tabelle 1, s. unten) amplifiziert und anschließend in den Vektor über die EcoR1- und Notl-Restriktionsschnittstellen kloniert (pET-32b(+)/calB_wt). Für die Subklonierung in pColdIII wurde CalB unter Verwendung der Primer wt_ pColdIII_fw und wt_pcoldIII_rev (Tabelle 1) amplifiziert und anschließend unter Verwendung von Standardverfahren in den Vektor kloniert (pColdIII/CalB_wt).

CalB_syn wurde aus pPCR/calB unter Verwendung der Primer syn_pUC18/pET-32b(+)_fw und syn_pUC18_rev, syn_pUC18/pET-32b(+)_fw und syn_pET-32b(+)_rev bzw. syn_pColdIII_fw und syn-pColdIII_rev (Tabelle 1) amplifiziert und anschließend in die Plasmide pUC18/calB_syn, pET-32b(+)/calB_syn und pColdIII/calB_syn kloniert, wobei Standardverfahren verwendet wurden, wodurch man die Konstrukte pUC18/calB_syn, pET-32b(+)/calB_syn und pColdIII/calB_syn erhielt.

Die nachfolgende Tabelle 1 zeigt die für die Subklonierung von calB-Varianten verwendeten Primer. Die Restriktionsschnittstellen sind unterstrichen.

**Tabelle 1**

| Primer | Sequenz | Restriktionsschnittstelle |
|---|---|---|
| wt-pUC18_fw | gatgaattcgctaccttccggttcggacc | *Eco*R1 |
| wt-pUC18_rev | ccacatatgtcagggggtgacgatgcc | *Nde*I |
| wt_pET-32b(+)_fw | ccggaattcgctaccttccggttc | *Eco*R1 |
| wt_pET-32b(+)_rev | cggcatcgtcaccccctaagcggccgc | *Not*I |
| wt_pColdIII_fw | cgattcatatgctaccttccggttcggacc | *Nde*I |
| wt_pColdIII_rev | ccttaagaattctcagggggtgacgatgcc | *Eco*R1 |
| syn_UC18/pET-32b(+)_fw | ccggaattcgctgccgagcgg | *Eco*R1 |
| syn_pUC18_rev | gtattgtgaccccgtaataacatatggaattcc | *Nde*I |
| syn_pET-32b(+)_rev | gcggtattgtgaccccgtaagcttggg | *Hind*III |
| syn_pColdIII_fw | cagttcatatgctgccgagcggtagcgat | *N*deI |
| syn_pColdIII_rev | ccttaagaattcttacggggtcacaataccgct | *Eco*R1 |

### Lipase-Expression und Co-Expression von Chaperonen

Expressionsexperimente wurden vier- bis sechsmal wiederholt und Aktivitäten als Mittelwerte angegeben.

### pUC18-Expression:

Origami™ B und DH5α-Zellen wurden mit pUC18-Konstrukten transformiert. Man ließ die Zellen bis zu einer optischen Dichte von 0,6 bei 600 nm bei 37 °C und 180 U/min in 100 ml LB-Medium (Luria 1960) wachsen, das 100 µg/ml Ampicillin enthielt (LBₐₘₚ). Anschließend wurde die Lipase-Expression durch Zugabe von IPTG induziert (Endkonzentration 1 mM). Man ließ die Zellen zusätzliche 4 Stunden bei 30 °C und 180 U/min wachsen und erntete durch 30minütige Zentrifugation mit 4000 g bei 4°C.

### pET-32b(+)-Expression:

Origami™ 2(DE3)-Zellen wurden mit pET32b(+)-Konstrukten transformiert. Man ließ die Zellen bei 37 °C und 180 U/min bis zu einer optischen Dichte von 0,6 bei 600 nm in 100 ml LBₐₘₚ wachsen und verarbeitete wie zuvor beschrieben. Alternativ wurden die Zellen vor der Induktion auf Eis gekühlt und die Expression 24 Stunden bei 15° C durchgeführt.

### pColdIII-Expression:

Origami™ B-Zellen wurden mit pColdIII-Konstrukten transformiert. Man ließ die Zellen bei 37 °C und 180 U/min bis zu einer optischen Dichte von 0,4-0,6 bei 600 nm in 100 ml LBₐₘₚ wachsen. Anschließend kühlte man die Kulturen 30 min auf Eis und induzierte die Lipase-Expression durch Zugabe von IPTG (Endkonzentration 1 mM). Man ließ die Zellen weitere 24 Stunden bei 15 °C und 180 U/min wachsen und erntete durch 30minütige Zentrifugation mit 4000 g bei 4°C.

### Co-Expression von Chaperon-Plasmiden und pColdIII-Konstrukten:

Origami™ B-Zellen wurden mit Chaperon-Plasmiden transformiert. Man ließ die Zellen bei 37 °C in 100 ml LB wachsen, das 34 µg/ml Chloramphenicol enthielt, und stellte über fachübliche Verfahren kompetente Zellen her. Die rekombinanten Zellen wurden mit den pColdIII-Konstrukten transformiert und auf LB_{cm+amp} selektiert. Für die Expression ließ man die Zellen bei 37 °C und 180 U/min bis zu einer optischen Dichte von 0,4-0,6 bei 600 nm in LB_{cm+amp} wachsen, das (im Fall von pGro7, pKJE7 und pTf16) 1 mg/ml L-Arabinose und (im Fall von pG-Tf2) 5 ng/ml Tetracyclin oder (im Fall von pG-KJE8) L-Arabinose und Tetracyclin in den oben angegebenen Konzentrationen enthielt. Die Kulturen wurden 30 min auf Eis gekühlt. Anschließend wurde die Expression von Lipase durch Zugabe von IPTG (Endkonzentration 1 mM) induziert. Man ließ die Zellen weitere 24 Stunden bei 15 °C und 180 U/min wachsen und erntete durch 30minütige Zentrifugation mit 4000 g bei 4°C.

### Tributyrin-Agarplatten-Assay

Zellen wurden auf LB-Agarplatten kultiviert, die 1 % emulgiertes Tributyrin und die entsprechenden Antibiotika sowie 1 mg/ml L-Arabinose bei Co-Expression von pGro7, pKJE7 oder pTf16, 5 ng/ml Tetracyclin bei Co-Expression von pG-Tf2, oder L-Arabinose und Tetracyclin bei Co-Expression von pG-KJE8 enthielten. Nach 24-stündigem Wachstum der Zellen bei 37 °C wurden die Platten mit Weichagar (0,6 % Agar in Wasser) überschichtet, der 1 mM IPTG enthielt, und für die Expression bei 30 °C, 15 °C oder Raumtemperatur inkubiert. Die Expression funktionaler Lipase wurde durch die Bildung klarer Halos um die Kolonien angezeigt.

### Lipase-Aktivitäts-Assay, SDS-PAGE und densitometrische Analyse

Man schloss die Zellen durch dreimalige Ultraschallbehandlung für eine Dauer von jeweils 30 sec in 50 mM Natriumphosphat-Puffer (pH 7,5) auf und entfernte Zelltrümmer durch Zentrifugation. Die Zell-Lysate wurden unter Verwendung der pH Stat-Vorrichtung (Metrohm, Filderstadt, Deutschland) auf Aktivität getestet. Die Hydrolyse des Substrats (5% Tributyrin, in Wasser mit 2 % Gummi arabicum emulgiert) wurde durch Titration mit 10 mM NaOH überwacht. Der Proteingehalt des Zelllysats wurde über den Bradford-Assay gemessen (Bradford 1976). Eine Lipase-Aktivitätseinheit wurde definiert als Freisetzung von 1 µmol Fettsäure pro Minute.
Unlösliche und lösliche Fraktionen wurden über SDS-PAGE nach Standardverfahren (Laemmli 1970) untersucht, wobei 50 µg des geklärten Zelllysats (entsprechend 0,2-0,4 ml Zellkultur) bzw. unlösliche Zelltrümmer aus 0,5 ml Zellkultur verwendet wurden. Die Gele wurden mit Coomassie Brilliant Blue gefärbt. Der Prozentsatz an löslichem CalB bezogen auf das gesamte Zellprotein wurde densitometrisch unter Verwendung des Programms "Scion Image" (Frederick, Maryland, USA) ermittelt.

### Hochdurchsatz-Expression und Aktivitäts-Assay

Man ließ pColdIII-Konstrukte und das Chaperon-Plasmid tragende Origami™ B-Zellen bis zu einer optischen Dichte von 0,4-0,6 bei 37 °C und 400 U/min in einer 96-Well-Mikrotiterplatte (Greiner, Nürtingen, Deutschland) wachsen, die 150 µl LB_{cm+amp} plus Chaperon-Induktor (siehe oben) enthielt. Die Zellen wurden 30 min auf Eis gekühlt und die Lipase-Expression durch Zugabe von IPTG auf eine Endkonzentration von 1 mM induziert. Nach 24-stündiger Expression bei 15 °C und Schütteln mit 200 U/min wurden die Zellen durch Zentrifugation geerntet und durch Zugabe von 50 µl Lysepuffer (50 mM Natriumphosphat pH 7,5, 1 mg/ml Lysozym, 1 µl/DNAse) lysiert. Die Lysate wurden bei 37 °C unter Schütteln (300 U/min) inkubiert und 30 min auf Eis gekühlt. Nach einstündiger Inkubation bei -80 °C wurden die Zellen bei Raumtemperatur (RT) aufgetaut und Zelltrümmer durch 30-minütige Zentrifugation mit 4000 U/min bei 4 °C entfernt.
Zum Nachweis von Lipaseaktivität gab man 20 µl geklärtes Zelllysat zu 180 µl Assay-Lösung (162 µl Lösung B (1 g Triton X-100 + 0,2 g Gummi arabicum in 200 ml 0,1 M Tris-HCl pH 7,5) + 18 µl Lösung A (60 mg pNPP in 20 ml n-Propanol). Die Bildung von p-Nitrophenolat wurde nach 5 min durch Messung der Extinktion bei 410 nm gemessen (Spectra Max 340PC, Molecular Devices Corp., Sunnyvale, CA, USA). Alternativ wurde die Lipase-Aktivität über den pH-Stat-Assay (wie oben beschrieben) quantifiziert.

### II. Ausführungsbeispiele

### 1. Klonierung von calB aus Candida antarctica

calB_wt-Gen ohne die für die N-terminale Prä-Pro-Peptidsequenz codierende Nukleotidsequenz wurde in die *E. coli*-Expressions-Vektoren pUC18, pET-32b(+) bzw. pColdIII kloniert (Tabelle 2). Das pPICZαA/caIB-Konstrukt (Rusnak 2004), welches das calB-Gen aus *Candida antarctica* enthielt, diente als Template für die Amplifizierung des Lipase-Gens.

Das calB-Gen wurde unter Verwendung der GeneOptimizer™-Technologie optimiert. Dabei wurde zusätzlich zur Optimierung der Codon-Präferenz wurden Regionen mit sehr hohem (> 80 %) oder niedrigem (< 30 %) GC-Gehalt falls möglich vermieden. Darüber hinaus wurden cis-wirkende Sequenzmotive wie beispielsweise interne TATA-Boxen, Chi-Stellen, ribosomale Andockungsstellen, ARE-, INS- und CRS-Sequenzelemente ebenso wie repetitive Sequenzen und RNA-Sekundärstrukturen vermieden. Das optimierte Gen (calB_syn) unterscheidet sich in 253 Nukleotiden (26,5%) von der calB_wt-Sequenz, die vorgenommenen Änderungen sind in Fig. 1 gezeigt. Auf Aminosäure-Ebene kodiert das synthetische Gen das veröffentlichte Protein (CAA83122.1). Anschließend wurde das synthetische Gen in die in Tabelle 2 erwähnten Expressionsvektoren subkloniert.

### Tabelle 2

**Tabelle 2: Verwendete Plasmide und Stämme. Pro: Promotor, Ori: Replikationsursprung**

| Plasmid | Gen von Interesse | Pro | Inducer | Ori | ResistenzMarker | Literaturnachweis |
|---|---|---|---|---|---|---|
| pPICZαA/calB | calB_wt | AOX1 | Methanol | pUC | Zeozin™ | (Invitrogen 2002) |
| pPCR/calB | calB_syn | / | / | ColE1 | Ampicillin | Geneart (Regensburg, Deutschland) |
| pUC18/calB | calB_wt / calB_syn | lac | IPTG | pBR322 | Ampicillin | MBI Fermentas (St. Leon-Rot, Deutschland) |
| pColdIII/calB | calB_wt / calB_syn | cspA | Kälteschock + IPTG | ColE1 | Ampicillin | (TaKaRa 2003) |
| pET32-b(+)/calB | Trx-calB_wt / Trx-calB_syn | T7 | IPTG | pBR322 | Ampicillin | (Novagen 1998) |
| pGro7 | groES-groEL | araB | *L*-Arabinose | pACYC | Chloramphenicol | (TaKaRa 2003) |
| pG-Tf2 | groES-groEL-tig | Pzt1 | Tetracyclin | pACYC | Chloramphenicol | (TaKaRa 2003) |
| pTf 16 | tig | araB | *L*-Arabinose | pACYC | Chloramphenicol | (TaKaRa 2003) |
| pKJE7 | dnaK-dnaJ-grpE | araB | *L*-Arabinose | pACYC | Chloramphenicol | (TaKaRa 2003) |
| pG-KJE8 | dnaK-dnaJ-grpE groES-groEL | araB Pzt1 | *L*-Arabinose Tetracyclin | pACYC | Chloramphenicol | (TaKaRa 2003) |
| | | | | | | |

| Stämme | Genotyp | | | | | Literaturnachweis |
|---|---|---|---|---|---|---|
| DH5α | supE44 ΔlacU169(Φ80lacZΔM15) hsdR17 recA1 end A1 gyrA96 thi1relA1 | | | | | Clontech (Heidelberg, Deutschland) |
| Origami™ B | Δara-leu7697 ΔlacX74 ΔphoAPvuII phoR araD139 ahpC galE galK rpsL F'[lac⁺(lacI^{q})pro] gor522::Tn10 (Tc^{R}) trxB::kan | | | | | (Novagen 2004) |
| Origami™ 2(DE3) | Δ(ara-leu)7697 ΔlacX74 ΔphoA PvuII phoR araD139 ahpC galE galK rpsL F'[lac+ lacI q pro] (DE3) gor522::Tn10 trxB (StrR, TetR) | | | | | (Novagen 2004) |

### 2. Lipase-Expression in drei unterschiedlichen Vektor-Systemen

Für die Expression von calB codierenden Vektoren wurden die Stämme *E. coli* Origami™ B und Origami™ 2(DE3) verwendet, die durch ihre Thioredoxin-Reduktase-und Glutathion-Reduktase-Defizienz charakterisiert sind. pUC18/calB_wt-transformierte Origami™ B-Zellen zeigten Halo-Bildung auf Tributyrin-Agarplatten, während dies beim Vergleichsstamm DH5α nicht der Fall war (Daten nicht gezeigt). Jedoch war die CalB-Aktivität in den mit pUC18/calB_wt oder pUC18/calB_syn transformierten Origami™ B-Zellen sehr niedrig und entsprach für beide Konstrukte einer Hydrolyse von nur etwa 2 U Tributyrin pro Milligramm gesamtes lösliches Protein (Fig. 4). 1 U (Unit) ist definiert als der Umsatz von 1 µmol Substrat pro Minute. In einer SDS-PAGE-Analyse wurde in der löslichen Fraktion keine Proteinbande nachgewiesen, die der Masse von CalB (33 kD) entsprach (Daten nicht gezeigt), während der CalB-Gehalt der unlöslichen Fraktion 10-12% betrug (Tabelle 3).

### Tabelle 3

**Tabelle 3: Densitometrische Analyse des CalB-Gehalts in Zellextrakten aus verschiedenen Expressionsexperimenten. Für die Auswertung des Coomassie Bluegefärbten SDS-PAGE-Gele wurde das Program Scion Image verwendet. N.d.: nicht nachweisbar.**

| | CalB-Gehalt der unlöslichen Fraktion [%] | | CalB-Gehalt der löslichen Fraktion [%] | | CalB-Gehalt des Gesamtzell-Proteins [%] | | Gehalt an löslichem CalB im Gesamtzell-Protein [%] | |
|---|---|---|---|---|---|---|---|---|
| | wt | syn | wt | syn | wt | syn | wt | syn |
| pUC18 | 12 | 10 | n.d. | n.d. | 4 | 3 | n.d. | n.d. |
| pET32-b(+) (30°C) | 19 | 18 | n.d. | n.d. | 6 | 6 | n.d. | n.d. |
| pET32-b(+) (15°C) | 24 | 26 | n.d. | n.d. | 8 | 9 | n.d. | n.d. |
| pColdIII | 42 | 38 | n.d. | n.d. | 14 | 13 | n.d. | n.d. |
| pColdIII + pGro7 | 49 | 28 | 11 | 10 | 23 | 16 | 7 | 7 |
| pColdIII + pG-Tf2 | 42 | 29 | 9 | 7 | 20 | 14 | 6 | 5 |
| pcoldIII + pTf16 | 22 | 19 | 10 | 5 | 14 | 9 | 7 | 3 |
| pcoldIII + pKJE7 | 31 | 15 | n.d. | n.d. | 10 | 5 | n.d. | n.d. |
| pcoldIII + pG-KJE8 | 5 | 6 | 4 | 4 | 4 | 5 | 3 | 3 |

Zur Erhöhung der Ausbeute an aktivem Enzym wurden die Gene calB_wt und calB_syn unter Verwendung des Vektors pET-32b(+) mit einem Thioredoxin-Tag (Trx •TAG™) fusioniert und in *E*.*coli* Origami™2 (DE3) exprimiert. Die transformierten Zellen zeigten bei Inkubation bei Raumtemperatur eine klare Halo-Bildung, während eine Kultivierung bei 37°C zu keiner nachweisbaren Enzymaktivität führte. Während die Expression von Trx-CalB in Schüttelkolbenkultur bei 30°C zu einem starken Anstieg der Enzymmenge in der unlöslichen Fraktion führte (18 - 19 %, Tabelle 3), nicht dagegen jedoch zu einem Anstieg der Löslichkeit und damit der Aktivität von CalB, ergab eine Expression bei 15°C bis zu 17 U/mg lösliches Protein beim wt-Gen (calB_wt) und 8 U/mg beim synthetischen Gen (calB_syn) (Fig. 4). In diesem System wurde weiterhin eine Halo-Bildung auf Tributyrin-Agarplatten nur bei niedrigen Kultivierungstemperaturen beobachtet. Trotzdem wurde durch SDS-PAGE eine hohe Menge an unlöslichem Protein bei Expression aus pET-32b(+) (24 % bzw. 26 %) und aus pcoldIII (42 % bzw. 38 %) nachgewiesen, selbst bei Expression bei 15 °C (Fig. 2, Tabelle 3).

### 3. Lipase-Expression mit Co-Expression molekularer Chaperone

Die pColdIII-Konstrukte gemäß Ausführungsbeispiel 2 wurden mit mehreren Kombinationen aus Chaperonen co-exprimiert, die im TaKaRa Chaperone Plasmid Set bereitgestellt werden (s. nachfolgende Tabelle 4).

### Tabelle 4

**Tabelle 4: Bestandteile des verwendeten Chaperon Plasmid Sets von TaKaRa**

| Plasmid | Chaperon | Promotor | Induktor | Resistenzmarker |
|---|---|---|---|---|
| pG-KJE8 | dnaK-dnaJ-grpE | araB | L-Arabinose | Cm |
| | groES-groEL | Pzt 1 | Tetracyclin | |
| pGro7 | groES-groEL | araB | L-Arabinose | Cm |
| pKJE7 | dnaK-dnaJ-grpE | araB | L-Arabinose | Cm |
| pG-Tf2 | groES-groEL | Pzt 1 | Tetracyclin | Cm |
| pTf 16 | Tig | araB | L-Arabinose | Cm |

Alle rekombinanten Origami™ B-Zellen, die sowohl CalB- als auch Chaperon-Expressionsplasmide trugen, zeigten bei einer Inkubationstemperatur von 15°C eine klare Halo-Bildung auf Tributyrin-Agarplatten und Expression von CalB im Schüttelkolbenmaßstab (Fig. 3), während sich die Menge von löslicher Lipase deutlich unterschied (Fig. 4). CalB_wt wurde am effizientesten bei Expression zusammen mit pGro7 (61 U/mg) exprimiert (Fig. 4). Die Expression von funktionalem Enzym wurde auch durch Expression zusammen mit pG-Tf2 (33 U/mg), pTf16 (24 U/mg) und, in geringerem Ausmaß, durch Expression zusammen mit pG-KJE8 (18 U/mg) gesteigert. Co-Expression zusammen mit pKJE7 beeinflusste die Expression von CalB nicht signifikant. Die Ergebnisse des Aktivitäts-Assay wurden densitometrisch bestätigt, wobei die größte Menge an löslichem CalB bei Co-Expression mit pGro7 gefunden wurde, gefolgt von Co-Expression mit pG-Tf2 und pTf16 (Tabelle 3).
Ähnliche Ergebnisse wurden mit dem synthetischen calB-Gen erhalten, wodurch ein positiver Einfluss der Co-Expression von pGro7, pG-Tf2, pTf16 und pG-KJE8 gezeigt wurde. Wie bei der pET-32b(+)- und der pColdIII-Expression waren die erhaltenen Werte für Lipase-Aktivitäten und Gehalt an löslichem Enzym beim synthetischen Gen calB_syn niedriger als beim Wildtyp-Gen calB_wt (Fig. 4, Tabelle 3).

### 4. Lipase-Expression auf Tributyrin-Agarplatten

GroES und GroEL (durch pGro7 kodiert) und CalB_wt bzw. CalB_syn exprimierende Origami™ B-Zellen zeigten bei Inkubation bei 15°C eine klare Halo-Bildung auf Tributyrin-supplementierten Agarplatten. Weder bei DH5α-Zellen, welche die gleichen Konstrukte enthielten, noch bei Origami™ B-Kontrollzellen, die pGro7-Vektor und pColdIII-Vektor ohne das Lipase-Gen enthielten, war Halo-Bildung zu beobachten (Daten nicht gezeigt).

### 5. Lipase-Expression im Mikrotiterplatten-Maßstab

Als Modell für ein Hochdurchsatz-Screening-System für von CalB abgeleitete Enzymvarianten wurde die Co-Expression von CalB_wt oder CalB_syn mit pGro7 in Origami™ B-Zellen in einem 96-Well-Mikrotiterplatten-Maßstab durchgeführt. Die Aktivitäten der geklärten Zelllysate wurden über einen kolorimetrischen Assay unter Verwendung von pNPP als Substrat quantifiziert. Wie durch die Bildung von gelbem p-Nitrophenolat gezeigt wurde (Figur 6), wurden beide Konstrukte in vergleichbaren Mengen funktional exprimiert. Kontrollzellen ohne das Lipase-Gen zeigten signifikant verringerte Extinktionswerte bei 410 nm.
Zum Vergleich der Aktivitäten bei Expression in Mikrotiterplatten mit den Werten, die bei Co-Expression der Konstrukte pColdIII/calB_wt and pGro7 in Schüttelkolben erhalten wurden, wurden die Aktivitäten von 5 repräsentativen Wells mit dem Substrat Tributyrin über den pH-Stat-Assay untersucht. Die Wachstumsbedingungen in den einzelnen Wells waren identisch. Als Ergebnis wurden spezifische Aktivitäten von 57 bis 81 U/mg lösliches Protein im geklärten Zelllysat bzw. Gesamtaktivitäten von 10 bis 15 U/ml Zellkultur ermittelt. Der Expressionsgrad an Gesamt-Lipase in den Wells wurde über densitometrische Analyse als 0,04±0,01 µg CalB/ml Zellkultur bestimmt.

### Literaturnachweis

Anderson E.M., Larsson, K.M., Kirk, O., Biocat. Biotransform. 16: 181-204 (1998)
Ausubel et al., Current Protocols in Molecular Biology, John Wiley & Sons, N.Y., 1989
Baneyx F., Mujacic M., Nature Biotechnol. 22(11): 1399-408 (2004)
Boel E., Huge-Jensen B., Christensen M., Thim L., Fill N.P., Lipids 23:701-706 (1988)
Bradford M.M., Anal. Biochem. 72: 248-254 (1976)
Chodrorge M., Fourage L., Ullmann C., Duvivier V., Masson J.M., Lefèvre F., Adv. Synth. Catal. 347: 1022-1026 (2005)
Choi E.-S., Sohn J.-H., Kim, S.-Y., WO 2004/024954 A1
Cooper T.G., "Biochemische Arbeitsmethoden", Walter de Gruyter-Verlag, Berlin, 1. Aufl., 1980, Seite 334-379
Gerritse G., Hommes R.W.J., Quax W.J., Appl. Environ. Microbiol. 64(7) 2644-2451 (1998)
Giacalone M.J., Gentile A.M., Lovitt B.T., Berkley N.L., Gunderson C.W., Surber M.W, Biotechniques 40(3): 355-364 (2006)
Giladi H., Goldenberg D., Koby S., Oppenheim A.B., Proc NatI Acad Sci USA 92:184-188 (1995)
Goeddel, Gene Expression Technology: Methods in Enzymology 185, Academic Press, San Diego, CA (1990)
Goldstein J., Pollitt N.S., Inouye M., Proc. NatI. Acad. Sci. USA 87(1): 283-7 (1990)
Hale R.S., Thompson, G., Protein Expr. Purif. 12: 185-188 (1988)
Hoegh I., Patkar, S., Halkier, T., Hansen, M.T., Can. J. Bot. 73: 869-875 (1995)
Ike Y, Ikuta S, Sato M, Huang T, Itakura K., Nucleic Acids Res. 11:477-488 (1983)
Ikura K., Kokubu T., Natsuka S., Ichikawa A., Adachi M., Nishihara K., Yanagi H., Utsumi S., Prep. Biochem. Biotechnol. 32(2): 189-205 (2002)
Itakura K, Rossi J.J., Wallace R.B., Annu. Rev. Biochem. 53:323-356 (1984)
Itakura K, Hirose T, Crea R, Riggs AD, Heyneker HL, Bolivar F, Boyer HW, Science 198:1056-1063 (1984)
Kirchhoff F., Desrosiers R.C., Methods Mol Biol. 57:323-33 (1996)
Laemmli U.K., Nature 227: 680-685 (1970)
Longhi S., Fusetti F., Grandori R., Lotti M., Vanoni M., Alberghina L., Biochim Biophys Acta 1131:227-232 (1992)
Lutz R., Bujard H., Nucleic Acids Res. 25(6): 1023-1210 (1997)
Martinelle M., Holmquist M., Hult K., Biochim. Biophys. Acta 1258(3): 272-6 (1995)
Nakashima K., Kanamaru K., Mizuno T., Horikoshi K., J. Bacteriol. 178:2994-2997 (1996)
Narang, S.A., Tetrahedron 39:3 (1983)
Nishihara K., Kanemori M., Yanagi H., Yura T., Appl. Environ. Microbiol. 66(3): 884-9 (2000)
Novagen (1999) pET Sytem Manual
Ollis D.L., Cheah, E., Cygler, M., Dijkstra, B., Frolow, F., Franken, S.M., Harel, M., Remingon, S.M., Silman, L., Schrag, J.D., Protein Eng. 5: 197-211 (1992).
Patkar S., Vind J., Kelstrup E., Christensen M.W., Svendsen A., Borch K., Kirk O., Chem. Phys. Lipids 93(1-2): 95-101 (1988)
Pearson W.R. und Lipman,D.J., Proc. NatI. Acad, Sci. (USA) 85(8): 2444-2448 (1988)
Prinz W.A., Aslund F., Holmgren A., Beckwith J. J. Biol. Chem. 272(25): 15661-7 (1997)
Qing G., Ma L.-C., Khorchid A., Swapna G.V.T., Mal T.K., Takayama M.M., Xia B., Phadtare S., Ke H., Acton T., Montelione G., Ikura M. und Inouye M., Nature Biotechnol. 22(7):877-882 (2004)
Qoronfleh M.W., Debouck C. Keller J., J. Bacteriol. 174:7902-7909 (1992)
Rotticci D."Understanding and Engineering the Enantioselectivity of Candida antarctica Lipase B towards sec-Alcohols". Stockholm: Royal institute of Technology 1-61 (2000)
Rotticci-Mulder J.C., "Expression and mutagenesis studies of Candida antarctica lipase B [PhD]". Stockholm: AlbaNova University Centre 1-74 (2003)
Rotticci-Mulder J.C., Gustavsson M., Holmquist M., Hult K., Martinelle M., Protein Expr. Purif. 21(3): 386-92 (2001)
Roufa D.J., Methods Mol. Biol. 57: 357-367 (1996)
Rusnak M. "Untersuchungen zur enzymatischen Enantiomerentrennung von Glykolethern und Etablierung neuer Methoden des synthetischen Shufflings". Universität Stuttgart (2004)
Rusnak M., Nieveler J., Schmid R.D., Petri R., Biotechnol Lett 27: 743-748 (2005)
Schmid R.D., Verger, R., Angew. Chem. Int. Ausg. 37: 1608-33 (1998)
Schofield D.A., Westwater C., Dolan J.W., Norris J.S., Schmidt M.G., Curr. Microbiol. 44(6): 425-430 (2002)
Scopes R. "Protein Purification. Prinicples and Practise". Springer Verlag, New York, 1. Aufl., 1981
Sharma R., Chisti Y., Banerjee U.C., Biotechnol Adv 19: 627-662 (2001)
Suen, W.C., Zhang, N., Xiao, L, Madison, V., Zaks, A. Protein Eng. Des. Sel. 17(2): 133-40 (2004)
TaKaRa (2003) Cold Shock Expression System pCold DNA, Manual 1-9
TaKaRa (2003b) Chaperone Plasmid Set Manual 1-7
Tsuchiya A., Nakazawa H., Toida J, Ohnishi K, Sekiguchi J, FEMS Microbiol Lett. 143:63-7 (1996)
Uppenberg J., Hansen, M.T., Patkar, S., Jones, A., Structure 2: 293-308 (1994)
Yamaguchi S., Mase T., Takeuchi K., Gene 103:61-67 (1991)
Zhang N., Suen W.C., Windsor W., Xiao L., Madison V., Zaks A., Protein Eng. 16(8): 599-605 (2003)

Folgende Sequenzen bzw. Plasmide, auf die in der vorangegangenen Beschreibung Bezug genommen wurde, sind im nachfolgenden Sequenzprotokoll unter den angegebenen SEQ ID NOs erfasst:
calB_syn: SEQ ID NO:1
calB_wt: SEQ ID NO:2
Promotor von T7: SEQ ID NO:3
cspA Promotor (*E.coli*): SEQ ID NO:4
calB_syn in pUC19 (pUC18/calB_syn): SEQ ID NO:5
calB_wt in pUC19 (pUC18/calB_swt): SEQ ID NO:6
calB_syn in pET-32b(+) (pET-32b(+)/calB_syn): SEQ ID NO:7
calB_wt in pET-32b(+) (pET-32b(+)/calB_wt): SEQ ID NO:8
calB_syn in pCOLDIII (pCOLDIII/calB_syn): SEQ ID NO:9
calB_wt in pCOLDIII (pCOLDIII/calB_wt): SEQ ID NO:10
pPCR/calB: SEQ ID NO:11
CalB (CAA83122.1): SEQ ID NO:12
CalB mit Austausch T57A und A89T: SEQ ID NO:13

## Patentansprüche

1. Verfahren zur Expression funktionaler Triacylglycerol-Lipase (E.C. 3.1.1.3) in Prokaryoten, **dadurch gekennzeichnet, dass** man eine Nukleotidsequenz gemäß SEQ ID NO:1 (calB_syn) oder SEQ ID NO:2 (calB_wt) oder eine dazu homologe Nukleotidsequenz, bei der nicht mehr als 20 % der Nukleotide verschieden sind von SEQ ID NO:1, wobei die Nukleotidsequenz eine Lipase B aus *Candida antarctica* (calB) codiert, unter der Kontrolle des induzierbaren Promotors von T7 (SEQ ID NO:3) oder unter der Kontrolle eines durch Kälteschock induzierbaren Promotors in einem Thioredoxin-Reduktase-defizienten und Glutathion-Reduktase-defizienten *E.coli*- Stamm bei einer Temperatur exprimiert, die ausgewählt ist aus einem Bereich von 1°C bis 25°C.

2. Verfahren nach Anspruch 1, **dadurch gekennzeichnet, dass** der durch Kälte induzierbare Promotor der Promotor des cspA-Gens von *E*.*co*/*i* (SEQ ID NO:4) ist.

3. Verfahren nach einem der Ansprüche 1 bis 2, **dadurch gekennzeichnet, dass** ein oder mehrere Chaperone co-exprimiert werden.

4. Verfahren nach Anspruch 3, **dadurch gekennzeichnet, dass** das co-exprimierte Chaperon ausgewählt ist bzw. die co-exprimierten Chaperone ausgewählt sind unter GroES, GroEL, DnaK, DnaJ, GrpE und Trigger Factor (TF) von *E.coli.*

5. Verfahren nach Anspruch 4, **dadurch gekennzeichnet, dass** GroEL und GroES co-exprimiert werden; oder dass DnaK, DnaJ und GrpE co-exprimiert werden; oder dass Trigger Factor, gegebenenfalls zusammen mit GroES und GroEL, co-exprimiert wird; oder dass DnaK, DnaJ, GrpE, GroES und GroEL co-exprimiert werden.

6. Verfahren zum Nachweis von Triacylglycerol-Lipasen, wobei man
i) ein Protein, für das man Triacylglycerol-Lipase-Aktivität vermutet, gemäß einem der Verfahren 1 bis 5 exprimiert,
ii) die Expressionsprodukt mit einem von Triacylglycerol-Lipase hydrolysierbaren Substrat in Kontakt bringt, und
iii) die Hydrolyseaktivität bestimmt.

7. Verfahren nach Anspruch 6, **dadurch gekennzeichnet, dass** die Expression des Proteins gemäß Schritt i) in Gegenwart eines von der Lipase hydrolysierbaren Substrats erfolgt.

8. Verfahren nach Anspruch 6, **dadurch gekennzeichnet, dass**
i) das exprimierte Protein aus der Zellkultur abgetrennt wird,
ii) mit einem von einer Triacylglycerol-Lipase hydrolysierbaren Substrat in Kontakt gebracht wird, und
iii) die Hydrolyseaktivität bestimmt wird.

9. Verwendung eines Verfahrens nach einem der Ansprüche 6 bis 8 zum Screenen mutagenisierter Proteine oder durch mutagenisierte Nukleinsäuren codierter Proteine auf Triacylglycerol-Lipase-Aktivität.

10. Verwendung nach Anspruch 9, **dadurch gekennzeichnet, dass** das Protein wenigstens eine Mutation in der codierende Nukleotidsequenz einer Lipase B aus *Candida antarctica* (calB) aufweist.

11. Verfahren oder Verwendung nach einem der Ansprüche 6 bis 10, **dadurch gekennzeichnet, dass** das Verfahren zum Nachweis von Triacylglycerol-Lipasen als Hochdurchsatz-Screening-Verfahren durchgeführt wird.

12. Verfahren zur Herstellung einer Triacylglycerol-Lipase (E.C. 3.1.1.3), **dadurch gekennzeichnet, dass** man ein mit Hilfe eines Verfahrens nach einem der Ansprüche 6 bis 11 ein exprimiertes Protein mit Aktivität einer Triacylglycerol-Lipase (E.C. 3.1.1.3) nachweist, den dieses Protein exprimierenden *E*.*coli*-Stamm unter Lipase-exprimierenden Bedingungen kultiviert und gegebenenfalls die exprimierte Lipase isoliert.

13. Für Triacylglycerol-Lipase codierende Nukleinsäure, **dadurch gekennzeichnet, dass** sie eine codierende Nukleotidsequenz gemäß SEQ ID NO:1 umfasst, oder eine zu dieser Referenzsequenz homologe Nukleotidsequenz umfasst, bei der nicht mehr als 20% der Nukleotide unterschiedlich sind.

14. Rekombinanter Vektor, **dadurch gekennzeichnet, dass** er eine codierende Nukleinsäure gemäß Anspruch 13 operativ verknüpft mit wenigstens einer regulativen Nukleinsäuresequenz umfasst.

15. Rekombinante Wirtszelle, **dadurch gekennzeichnet, dass** sie eine Nukleinsäure gemäß Anspruch 13 und/oder einen rekombinanten Vektor gemäß Anspruch 14 umfasst.

16. Verwendung einer codierenden Nukleinsäuresequenz nach Anspruch 13, eines Vektors nach Anspruch 14 oder einer Wirtszelle nach Anspruch 15 zur Durchführung eines Verfahrens nach einem der Ansprüche 1 bis 12.

## Claims

1. A method of expression of a functional triacylglycerol lipase (E.C. 3.1.1.3) in prokaryotes, wherein a nucleotide sequence as claimed in SEQ ID NO:1 (calB_syn) or SEQ ID NO:2 (calB_wt) or a nucleotide sequence homologous thereto, in which not more than 20% of the nucleotides are different from SEQ ID NO:1, the nucleotide sequence encoding a lipase B from *Candida antarctica* (calB), is expressed in a thioredoxin-reductase-deficient and glutathione-reductase-deficient *E.coli* strain under the control of the inducible promoter of T7 (SEQ ID NO:3) or under the control of a cold-shock-inducible promoter at a temperature which is selected from a range from 1°C to 25°C.

2. The method as claimed in claim 1, wherein the cold-inducible promoter is the promoter of the cspA gene of *E.coli* (SEQ ID NO:4).

3. The method as claimed in one of claims 1 to 2, wherein one or more chaperones are co-expressed.

4. The method as claimed in claim 3, wherein the co-expressed chaperone is selected or the co-expressed chaperones are selected from GroES, GroEL, DnaK, DnaJ, GrpE and Trigger Factor (TF) of *E.coli.*

5. The method as claimed in claim 4, wherein GroEL and GroES are co-expressed; or wherein DnaK, DnaJ and GrpE are co-expressed; or wherein Trigger Factor, optionally together with GroES and GroEL, is co-expressed; or wherein DnaK, DnaJ, GrpE, GroES and GroEL are co-expressed.

6. A method for the detection of triacylglycerol lipases, wherein
i) a protein that is presumed to have triacylglycerol lipase activity is expressed as claimed in one of the methods 1 to 5,
ii) the expression product is brought into contact with a substrate that is hydrolyzable by triacylglycerol lipase, and
iii)the hydrolysis activity is determined.

7. The method as claimed in claim 6, wherein expression of the protein as claimed in step i) takes place in the presence of a substrate that is hydrolyzable by the lipase.

8. The method as claimed in claim 6, wherein
i) the expressed protein is separated from the cell culture,
ii) is contacted with a substrate that is hydrolyzable by a triacylglycerol lipase, and
iii)the hydrolysis activity is determined.

9. The use of a method as claimed in one of claims 6 to 8 for screening mutagenized proteins or proteins encoded by mutagenized nucleic acids for triacylglycerol lipase activity.

10. The use as claimed in claim 9, wherein the protein has at least one mutation in the coding nucleotide sequence of a lipase B from *Candida antarctica* (calB).

11. The method or the use as claimed in one of claims 6 to 10, wherein the method is carried out for the detection of triacylglycerol lipases as a high-throughput screening method.

12. A method of production of a triacylglycerol lipase (E.C. 3.1.1.3), wherein a protein expressed using a method as claimed in one of claims 6 to 11 with activity of a triacylglycerol lipase (E.C. 3.1.1.3) is detected, the *E.coli* strain expressing this protein is cultivated under lipase-expressing conditions and optionally the expressed lipase is isolated.

13. A nucleic acid encoding triacylglycerol lipase, wherein it comprises a coding nucleotide sequence as claimed in SEQ ID NO:1, or comprises a nucleotide sequence homologous to said reference sequence, in which not more than 20% of the nucleotides are different.

14. A recombinant vector, wherein it comprises a coding nucleic acid as claimed in claim 13 operatively linked to at least one regulating nucleic acid sequence.

15. A recombinant host cell, wherein it comprises a nucleic acid as claimed in claim 13 and/or a recombinant vector as claimed in claim 14.

16. The use of a coding nucleic acid sequence as claimed in claim 13, of a vector as claimed in claim 14 or of a host cell as claimed in claim 15 for carrying out a method as claimed in one of claims 1 to 12.

## Revendications

1. Procédé pour l'expression de triacylglycérol-lipase fonctionnelle (E.C. 3.1.1.3) dans des procaryotes, **caractérisé en ce qu'**on exprime une séquence nucléotidique selon SEQ ID NO:1 (calB_syn) ou SEQ ID NO:2 (calB_wt), ou une séquence nucléotidique qui est homologue de cette dernière, dans laquelle pas plus de 20 % des nucléotides sont différents de SEQ ID NO:1, la séquence nucléotidique codant pour une lipase B de *Candida antarctica* (calB), sous le contrôle du promoteur inductible de T7 (SEQ ID NO:3) ou sous le contrôle d'un promoteur inductible par un choc froid, dans une souche de *E. coli* déficiente en thiorédoxine-réductase et déficiente en glutathion-réductase, à une température qui est choisie dans une plage de 1 à 25°C.

2. Procédé selon la revendication 1, **caractérisé en ce que** le promoteur inductible par le froid est le promoteur du gène cspA de *E. coli* (SEQ ID NO:4).

3. Procédé selon l'une des revendications 1 à 2, **caractérisé en ce qu'**un ou plusieurs chaperons sont co-exprimés.

4. Procédé selon la revendication 3, dans lequel le chaperon co-exprimé ou les chaperons co-exprimés sont choisis parmi GroES, GroEL, DnaK, DnaJ, GrpE et le facteur déclenchant (TF) de *E. coli.*

5. Procédé selon la revendication 4, **caractérisé en ce que** GroEL et GroES sont co-exprimés, ou que DnaK, DnaJ et GrpE sont co-exprimés, ou que le facteur déclenchant est co-exprimé, éventuellement en même temps que GroES et GroEL, ou que DnaK, DnaJ, GrpE, GroES et GroEL sont co-exprimés.

6. Procédé pour la détection de triacylglycérol-lipases, dans lequel
i) on exprime selon l'un des procédés 1 à 5 une protéine pour laquelle on suspecte une activité de triacylglycérol-lipase,
ii) on met en contact le produit de l'expression avec un substrat hydrolysable par la triacylglycérol-lipase, et
iii) on détermine l'activité d'hydrolyse.

7. Procédé selon la revendication 6, **caractérisé en ce que** l'expression de la protéine s'effectue selon l'étape i) en présence d'un substrat hydrolysable par la lipase.

8. Procédé selon la revendication 6, **caractérisé en ce que**,
i) on sépare la protéine exprimée de la culture cellulaire,
ii) on la met en contact avec un substrat hydrolysable par une triacylglycérol-lipase, et
iii) on détermine l'activité d'hydrolyse.

9. Utilisation d'un procédé selon l'une des revendications 6 à 8 pour cribler pour l'activité de trigacylglycérol-lipase des protéines mutagénisées ou des protéines codées par des acides nucléiques mutagénisés.

10. Utilisation selon la revendication 9, **caractérisée en ce que** la protéine présente au moins une mutation dans la séquence nucléotidique codante d'une lipase de *Candida antarctica* (calB).

11. Procédé ou utilisation selon l'une des revendications 6 à 10, **caractérisé en ce que** le procédé pour la détection de triacylglycérol-lipases est mis en oeuvre sous forme d'un procédé de criblage à haut débit.

12. Procédé de préparation d'une triacylglycérol-lipase (E.C. 3.1.1.3), **caractérisé en ce qu'**on détecte, à l'aide d'un procédé selon l'une des revendications 6 à 11, une protéine exprimée ayant l'activité d'une triacylglycérol-lipase (E.C. 3.1.1.3), on cultive la souche de *E. coli* exprimant cette protéine dans des conditions permettant l'expression d'une lipase, et éventuellement on isole la lipase exprimée.

13. Acide nucléique codant pour la triacylglycérol-lipase, **caractérisé en ce qu'**il comprend une séquence nucléotidique codante selon SEQ ID NO:1, ou comprend une séquence nucléotidique homologue de cette séquence de référence, dans laquelle pas plus de 20 % des nucléotides sont différents.

14. Vecteur recombinant, **caractérisé en ce qu'**il comprend un acide nucléique codant selon la revendication 13, fonctionnellement lié à au moins une séquence d'acide nucléique régulatrice.

15. Cellule hôte recombinante, **caractérisée en ce qu'**elle comprend un acide nucléique selon la revendication 13 et/ou un vecteur recombinant selon la revendication 14.

16. Utilisation d'une séquence d'acide nucléique codante selon la revendication 13, d'un vecteur selon la revendication 14 ou d'une cellule hôte selon la revendication 15 pour la mise en oeuvre d'un procédé selon l'une des revendications 1 à 12.
